# EUROPEAN PATENT APPLICATION

(11) **EP 4 491 626 A1**
(43) Date of publication of application: **15.01.2025**
(21) Application number: 23820100.8
(22) Date of filing: 08.06.2023
(51) Int. Cl.: C07D 471/04, A61K 31/519, A61P 35/00, A23L 33/10

(54) **PHENYLSULFONAMIDE DERIVATIVES, PREPARATION METHOD THEREFOR, AND PHARMACEUTICAL COMPOSITION FOR PREVENTION OR TREATMENT OF CANCER CONTAINING SAME AS ACTIVE INGREDIENT**

(30) Priority: 08.06.2022 KR 20220069624; 07.06.2023 KR 20230073081
(71) Applicant: Korea Research Institute of Chemical Technology, Daejeon 34114 (KR); Aston Sci. Inc., Seoul 06193 (KR)
(72) Inventor: HEO, Jung Nyoung, Daejeon 34114 (KR); KIM, Seong Hwan, Daejeon 34114 (KR); YOON, Eun Young, Daejeon 34114 (KR); KIM, Eun Hye, Daejeon 34114 (KR); LEE, Yun O, Daejeon 34114 (KR); CHA, Hyeon Min, Daejeon 34114 (KR); PARK, Sun Gil, Daejeon 34114 (KR); JUNG, Hun, Seoul 06193 (KR); PARK, Hyo-Hyun, Seoul 06193 (KR); LIM, Jin Back, Seoul 06193 (KR); CHOI, Youngki, Seoul 06193 (KR)
(74) Representative: Office Freylinger
(86) International application number: PCT/KR2023/007798
(87) International publication number: WO 2023/239165

(57) **Abstract**

The present invention relates to: phenylsulfonamide derivatives; a preparation method therefor; and a pharmaceutical composition containing same as an active ingredient for the prevention or treatment of cancer. The compound represented by chemical formula 1 disclosed in the present specification exhibited excellent inhibitory effects against kinases related to integrated stress response (ISR) and is thus expected to be useful as a treatment for cancer.

## Description

### BACKGROUND OF THE INVENTION

### 1. Field of the Invention

The present invention relates to a phenylsulfonamide derivative, a preparation method thereof, and a pharmaceutical composition containing the same as an active ingredient for the prevention or treatment of cancer.

### 2. Description of the Related Art

Millions of people worldwide die from a variety of cancers, including bone, bladder, blood (leukemia), brain, breast, colon, cervical, esophageal, bowel, kidney, lung, liver, oral, nasal, nerve, ovarian, pancreatic, skin, stomach, prostate, throat, uterine, and vaginal cancers.

Over the years, various methods including radiotherapy and chemotherapy have been used to treat cancer, but the number of cancer patients is still increasing, and the side effects of the radiotherapy and chemotherapy, which cause illness and even death in some patients, are widely reported.

One of the most fundamental characteristics of cancer cells is their adaptation to cellular stress. Dysregulation of integrated stress response (ISR) has been linked to various types of diseases, including cancer. In addition, various stresses are sensed by four specialized kinases (PERK, GCN2, PKR, and HRI) that converge on the phosphorylation of serine 51 of eIF2α. Thus, pharmacologic regulation of ISR is known to be closely related to cancer treatment (Tian, Xiaobing, et al. Frontiers in Pharmacology (2021): 2621). PERK is an anticancer target protein that induces cancer survival, growth, and metastasis, and is also known to be involved in anticancer drug resistance (Bu, Yiwen et al. Journal of cellular physiology 231.10 (2016): 2088-2096). In addition, the expression of GCN is associated with the prognosis of cancer patients, and it is known to play a critical role in the survival and proliferation of cancer cells (Ge Liyuan et al. Cancer Biomarkers 22.3 (2018): 395-403).

Accordingly, the present inventors discovered that the phenylsulfonamide derivative inhibits kinase signaling involved in ISR and thus has utility as an anticancer agent, thereby completing the present invention.

### SUMMARY OF THE INVENTION

It is an object of the present invention to provide a novel phenylsulfonamide derivative.

It is another object of the present invention to provide a preparation method of a novel phenylsulfonamide derivative.

It is another object of the present invention to provide a pharmaceutical composition for the prevention or treatment of cancer.

It is another object of the present invention to provide a pharmaceutical composition for the prevention or treatment of a disease related to ISR (integrated stress response).

It is another object of the present invention to provide a health functional food for the prevention or amelioration of cancer.

To achieve the above objects, in an aspect of the present invention, the present invention provides a compound represented by formula 1 below, a stereoisomer thereof, a solvate thereof, a hydrate thereof, or a pharmaceutically acceptable salt thereof.

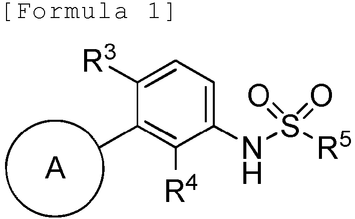

In formula 1, A is 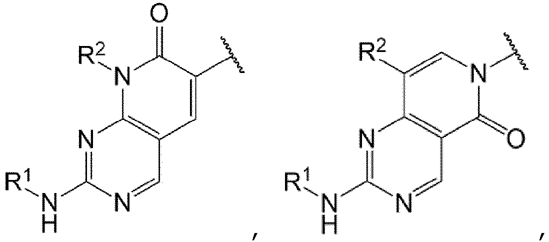 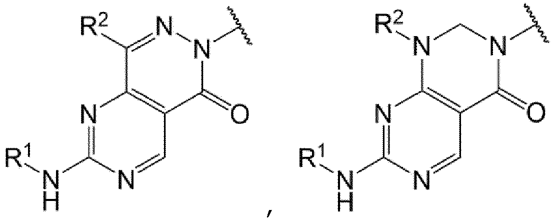 or 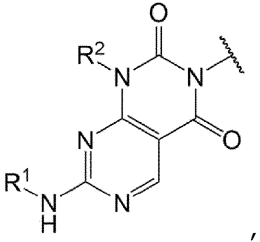
R¹ is hydrogen or C1-C10 alkyl;
R² is hydrogen, halogen, C1-C10 alkyl unsubstituted or substituted with halogen or hydroxyl, or C3-C10 cycloalkyl;
R³ and R⁴ are independently halogen;
R⁵ is unsubstituted or substituted C6-C10 aryl, or unsubstituted or substituted 5-10 membered heteroaryl,
wherein, the substituted C6-C10 aryl and the substituted 5-10 membered heteroaryl are independently substituted with at least one selected from the group consisting of C1-C10 alkyl unsubstituted or substituted with halogen or hydroxyl, C1-C10 alkoxy, halogen, and cyano, or substituted for two adjacent atoms to form 5-6 membered heterocycle. At this time, the 5-6 membered heterocycle contains at least one N, and is unsubstituted or substituted with at least one selected from C1-C10 alkyl and oxo.

In another aspect of the present invention, the present invention provides a preparation method of a compound represented by formula 1, comprising a step of reacting a compound represented by formula 2 with a compound represented by formula 3 to prepare a compound represented by formula 1, as shown in reaction formula 1 below.

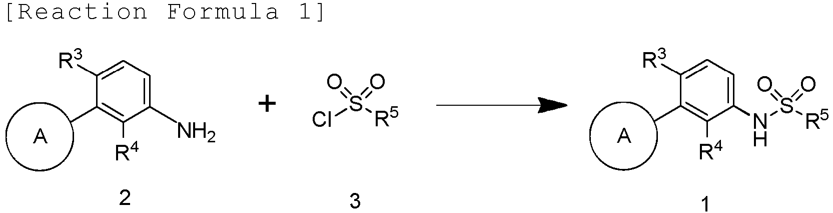

In reaction formula 1,
A, R¹, R², R³, R⁴, and R⁵ are as defined in formula 1 above.

In another aspect of the present invention, the present invention provides a pharmaceutical composition for the prevention or treatment of cancer, comprising a compound represented by formula 1 disclosed in the present specification, a stereoisomer thereof, a solvate thereof, a hydrate thereof, or a pharmaceutically acceptable salt thereof as an active ingredient.

In another aspect of the present invention, the present invention provides a pharmaceutical composition for the prevention or treatment of a disease caused by overactivation of kinases involved in ISR (integrated stress response), comprising a compound represented by formula 1 disclosed in the present specification, a stereoisomer thereof, a solvate thereof, a hydrate thereof, or a pharmaceutically acceptable salt thereof as an active ingredient.

In another aspect of the present invention, the present invention provides a health functional food for the prevention or amelioration of cancer, comprising a compound represented by formula 1 disclosed in the present specification, a stereoisomer thereof, a solvate thereof, a hydrate thereof, or a pharmaceutically acceptable salt thereof as an active ingredient.

### ADVANTAGEOUS EFFECT

The compound represented by chemical formula 1 disclosed in the present specification exhibited excellent inhibitory effects against kinases related to integrated stress response (ISR) and is thus expected to be useful as a treatment for cancer.

### BRIEF DESCRIPTION OF THE DRAWINGS

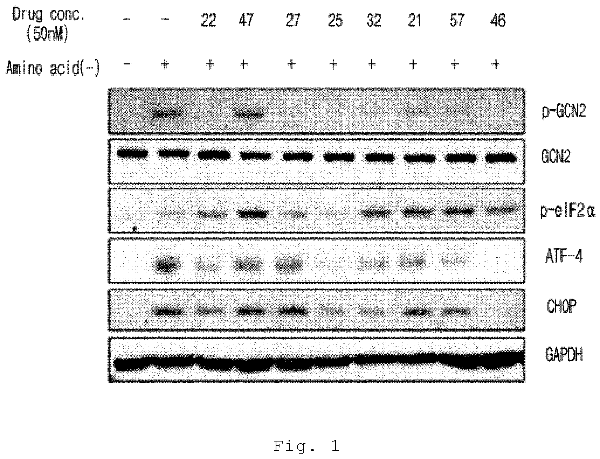

Figure 1 is a set of photographs confirming the mechanism proteins that respond upon ISR activation, wherein the numbers correspond to example numbers of the present invention.

### DESCRIPTION OF THE PREFERRED EMBODIMENTS

Hereinafter, the present invention is described in detail.

The embodiments of this invention can be modified in various other forms, and the scope of the present invention is not limited to the embodiments described below. It is well understood by those in the art who has the average knowledge on this field that the embodiments of the present invention are given to explain the present invention more precisely.

In addition, the "inclusion" of an element throughout the specification does not exclude other elements, but may include other elements, unless specifically stated otherwise.

The term "alkylene," "alkenyl," or "alkyl" includes straight or branched hydrocarbon residues, unless otherwise specified. For example, "C1-C5 alkyl" refers to alkyl having a skeleton of 1 to 5 carbons. Specifically, C1-C5 alkyl can include methyl, ethyl, n-propyl, i-propyl, n-butyl, i-butyl, t-butyl, n-pentyl, i-pentyl, t-pentyl, sec-pentyl, neopentyl, and the like.

The term "cycloalkyl" includes a carbocyclic group containing carbon atoms, unless otherwise specified. For example, "C3-C8 cycloalkyl" refers to cycloalkyl having a skeleton of 3 to 8 carbons. Specifically, C3-C8 cycloalkyl can include cyclopropyl, cyclobutyl, cyclopentyl, cyclohexyl, and the like.

The term "aryl" refers to a functional group in which one hydrogen atom has been removed from aromatic hydrocarbon, and includes single rings and double or more fused aromatic hydrocarbon functional groups. For example, C6-C10 aryl refers to a fully unsaturated hydrocarbon single or fused double ring compound having 6 to 10 carbon atoms that is fully unsaturated and satisfies Huckel's rule, and includes phenyl and naphthyl groups.

The term "heterocycle" includes fully saturated heterocycloalkyl or partially unsaturated heterocycloalkyl and heteroaryl.

The term "heterocycloalkyl", unless otherwise specified, includes a monovalent saturated residue consisting of 1 to 3 rings containing 1, 2, 3 or 4 heteroatoms selected from N, O or S. Two or three rings may contain bridged, fused or spiro heterocycloalkyls. And, 5-6 membered heterocycloalkyl refers to heterocycloalkyl with either 5 or 6 atoms forming the ring.

The term "heteroaryl" may include an aromatic radical with a single ring or two or three fused rings having one or more aromatic rings containing 1, 2 or 3 ring heteroatoms selected from N, O or S, unless otherwise specified. And, 5-6 membered heteroaryl refers to heteroaryl with either 5 or 6 atoms forming the ring.

The term "halogen" refers to a monovalent substituent group including F, Cl, Br, and I.

In one embodiment of the present invention, the present invention provides a compound represented by formula 1 below, a stereoisomer thereof, a solvate thereof, a hydrate thereof, or a pharmaceutically acceptable salt thereof.

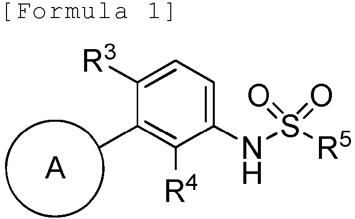

In formula 1,
A is 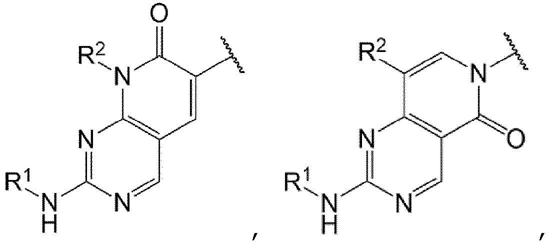 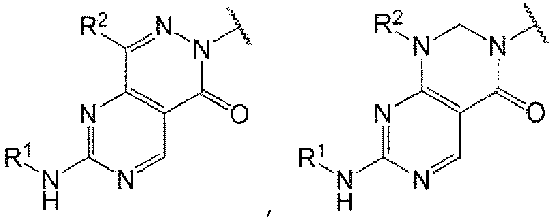 or 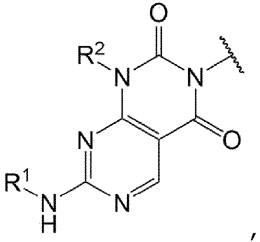
   R¹ is hydrogen or C1-C10 alkyl;
   R² is hydrogen, halogen, C1-C10 alkyl unsubstituted or substituted with halogen or hydroxyl, or C3-C10 cycloalkyl;
   R³ and R⁴ are independently halogen;
   R⁵ is unsubstituted or substituted C6-C10 aryl, or unsubstituted or substituted 5-10 membered heteroaryl,
   wherein, the substituted C6-C10 aryl and the substituted 5-10 membered heteroaryl are independently substituted with at least one selected from the group consisting of C1-C10 alkyl unsubstituted or substituted with halogen or hydroxyl, C1-C10 alkoxy, halogen, and cyano, or substituted for two adjacent atoms to form 5-6 membered heterocycle. At this time, the 5-6 membered heterocycle contains at least one N, and is unsubstituted or substituted with at least one selected from C1-C10 alkyl and oxo.

In one embodiment of the present invention, the present invention provides a compound of formula 1, a stereoisomer thereof, a solvate thereof, a hydrate thereof, or a pharmaceutically acceptable salt thereof wherein:
R¹ is hydrogen or C1-C6 alkyl;
R² is hydrogen, halogen, C1-C6 alkyl unsubstituted or substituted with 1 to 3 halogens or hydroxyls, or C3-C8 cycloalkyl;
R³ and R⁴ are independently halogen;
R⁵ is unsubstituted or substituted C6-C10 aryl, or unsubstituted or substituted 5-9 membered heteroaryl,
wherein, the substituted C6-C10 aryl and the substituted 5-9 membered heteroaryl are independently substituted with 1 to 3 substituents selected from the group consisting of C1-C6 alkyl unsubstituted or substituted with 1 to 3 halogens or hydroxyls, C1-C6 alkoxy, halogen, and cyano, or substituted for two adjacent atoms to form 5-6 membered heterocycle. At this time, the 5-6 membered heterocycle contains at least one N, and is unsubstituted or substituted with 1 to 3 substituents selected from C1-C6 alkyl and oxo.

In one embodiment of the present invention, the present invention provides a compound of formula 1, a stereoisomer thereof, a solvate thereof, a hydrate thereof, or a pharmaceutically acceptable salt thereof wherein:
R¹ is hydrogen or C1-C4 alkyl;
R² is hydrogen, halogen, C1-C4 alkyl unsubstituted or substituted with 1 to 3 halogens or hydroxyls, or C3-C6 cycloalkyl;
R³ and R⁴ are independently halogen;
R⁵ is unsubstituted or substituted C6-C10 aryl, or unsubstituted or substituted 5-9 membered heteroaryl,
wherein, the substituted C6-C10 aryl and the substituted 5-9 membered heteroaryl are independently substituted with 1 to 3 substituents selected from the group consisting of C1-C4 alkyl unsubstituted or substituted with 1 to 3 Fs or hydroxyls, C1-C4 alkoxy, F, Cl, Br, and cyano, or substituted for two adjacent atoms to form 5 membered non-aromatic heterocycle. At this time, the 5 membered heterocycle contains at least one N, and is unsubstituted or substituted with 1 to 2 substituents selected from C1-C4 alkyl and oxo.

In one embodiment of the present invention, the present invention provides a compound of formula 1, a stereoisomer thereof, a solvate thereof, a hydrate thereof, or a pharmaceutically acceptable salt thereof wherein:
R¹ is hydrogen or methyl;
R² is hydrogen, halogen, methyl unsubstituted or substituted with 1 to 3 Fs or hydroxyls, or cyclopropyl;
R³ and R⁴ are independently F;
R⁵ is substituted phenyl, naphthalenyl, substituted pyridinyl, substituted pyrazolyl, substituted isoxazolyl, substituted thiophenyl, substituted indolyl, or substituted imidazothiazolyl,
wherein, the substituted phenyl, substituted pyridinyl, substituted pyrazolyl, substituted isoxazolyl, substituted thiophenyl, substituted indolyl, and substituted imidazothiazolyl are independently substituted with 1 to 3 substituents selected from the group consisting of methyl unsubstituted or substituted with 1 to 3 Fs or hydroxyls, ethyl, methoxy, F, Cl, Br, oxo, and cyano, or substituted for two adjacent atoms to form 5 membered non-aromatic heterocycle. At this time, the 5 membered heterocycle contains at least one N, and is substituted with 1 to 2 substituents selected from methyl and oxo.

In one embodiment of the present invention, the present invention provides a compound of formula 1, a stereoisomer thereof, a solvate thereof, a hydrate thereof, or a pharmaceutically acceptable salt thereof wherein:
R¹ is hydrogen or methyl;
R² is hydrogen, 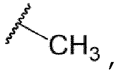 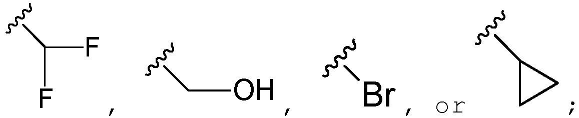
R⁵ is 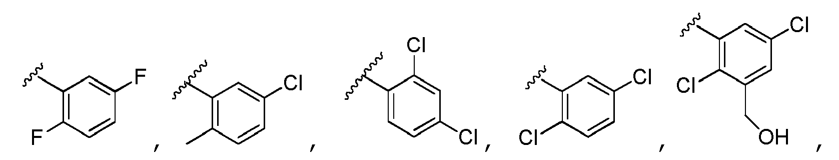 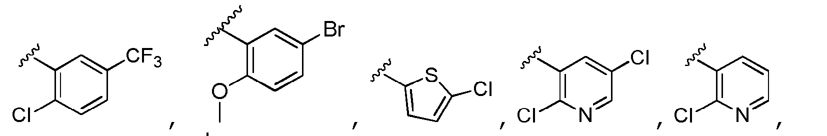 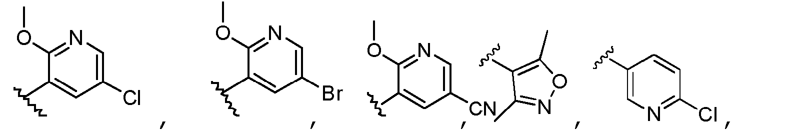 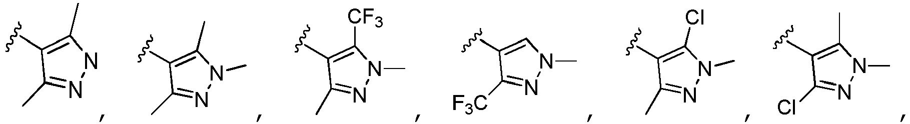 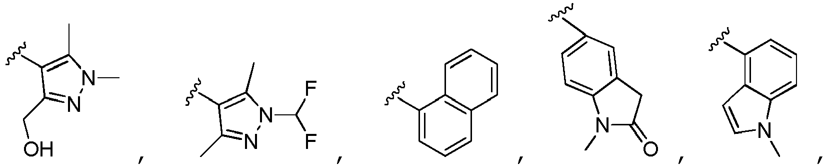 or

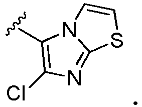

In one embodiment of the present invention, the present invention provides a compound of formula 1, a stereoisomer thereof, a solvate thereof, a hydrate thereof, or a pharmaceutically acceptable salt thereof wherein the compound represented by formula 1 above is one selected from the following group of compounds.
<1> N-(3-(2-amino-8-methyl-7-oxo-7,8-dihydropyrido[2,3-d]pyrimidin-6-yl)-2,4-difluorophenyl)-1,3-dimethyl-5-(trifluoromethyl)-1H-pyrazole-4-sulfonamide;
<2> N-(3-(2-amino-8-methyl-7-oxo-7,8-dihydropyrido[2,3-d]pyrimidin-6-yl)-2,4-difluorophenyl)-5-chloro-1,3-dimethyl-1H-pyrazole-4-sulfonamide;
<3> N-(3-(2-amino-8-methyl-7-oxo-7,8-dihydropyrido[2,3-d]pyrimidin-6-yl)-2,4-difluorophenyl)-1,3,5-trimethyl-1H-pyrazole-4-sulfonamide;
<4> N-(3-(2-amino-8-methyl-7-oxo-7,8-dihydropyrido[2,3-d]pyrimidin-6-yl)-2,4-difluorophenyl)-5-chloro-2-methoxypyridine-3-sulfonamide;
<5> N-(3-(2-amino-8-methyl-7-oxo-7,8-dihydropyrido[2,3-d]pyrimidin-6-yl)-2,4-difluorophenyl)-3,5-dimethyl-1H-pyrazole-4-sulfonamide;
<6> N-(3-(2-amino-8-methyl-7-oxo-7,8-dihydropyrido[2,3-d]pyrimidin-6-yl)-2,4-difluorophenyl)-6-chloropyridine-3-sulfonamide;
<7> N-(3-(2-amino-8-methyl-7-oxo-7,8-dihydropyrido[2,3-d]pyrimidin-6-yl)-2,4-difluorophenyl)-1-methyl-1H-indole-4-sulfonamide;
<8> N-(3-(2-amino-8-methyl-7-oxo-7,8-dihydropyrido[2,3-d]pyrimidin-6-yl)-2,4-difluorophenyl)-1-(difluoromethyl)-3,5-dimethyl-1H-pyrazole-4-sulfonamide;
<9> N-(3-(2-amino-8-methyl-7-oxo-7,8-dihydropyrido[2,3-d]pyrimidin-6-yl)-2,4-difluorophenyl)-3-chloro-1,5-dimethyl-1H-pyrazole-4-sulfonamide;
<10> N-(3-(2-amino-8-methyl-7-oxo-7,8-dihydropyrido[2,3-d]pyrimidin-6-yl)-2,4-difluorophenyl)-3-(hydroxymethyl)-1,5-dimethyl-1H-pyrazole-4-sulfonamide;
<11> N-(3-(2-amino-8-methyl-7-oxo-7,8-dihydropyrido[2,3-d]pyrimidin-6-yl)-2,4-difluorophenyl)-2-chloropyridine-3-sulfonamide;
<12> N-(3-(2-amino-8-methyl-7-oxo-7,8-dihydropyrido[2,3-d]pyrimidin-6-yl)-2,4-difluorophenyl)-2,5-difluorobenzene sulfonamide;
<13> N-(3-(2-amino-8-methyl-7-oxo-7,8-dihydropyrido[2,3-d]pyrimidin-6-yl)-2,4-difluorophenyl)-3,5-dimethylisoxazole-4-sulfonamide;
<14> N-(3-(2-amino-8-methyl-7-oxo-7,8-dihydropyrido[2,3-d]pyrimidin-6-yl)-2,4-difluorophenyl)-2,5-dichlorobenzenesulfonamide;
<15> N-(3-(2-amino-8-methyl-7-oxo-7,8-dihydropyrido[2,3-d]pyrimidin-6-yl)-2,4-difluorophenyl)-6-chloroimidazo[2,1-b]thiazole-5-sulfonamide;
<16> N-(3-(2-amino-8-methyl-7-oxo-7,8-dihydropyrido[2,3-d]pyrimidin-6-yl)-2,4-difluorophenyl)naphthalene-1-sulfonamide;
<17> N-(3-(2-amino-8-methyl-7-oxo-7,8-dihydropyrido[2,3-d]pyrimidin-6-yl)-2,4-difluorophenyl)-1-methyl-3-(trifluoromethyl)-1H-pyrazole-4-sulfonamide;
<18> N-(3-(2-amino-8-methyl-7-oxo-7,8-dihydropyrido[2,3-d]pyrimidin-6-yl)-2,4-difluorophenyl)-2-chloro-5-(trifluoromethyl)benzenesulfonamide;
<19> N-(3-(2-amino-8-methyl-7-oxo-7,8-dihydropyrido[2,3-d]pyrimidin-6-yl)-2,4-difluorophenyl)-5-chlorothiophene-2-sulfonamide;
<20> 2,5-dichloro-N-(2,4-difluoro-3-(8-methyl-2-(methylamino)-7-oxo-7,8-dihydropyrido[2,3-d]pyrimidin-6-yl)phenyl)benzenesulfonamide;
<21> N-(3-(2-amino-8-methyl-5-oxopirido[4,3-d]pyrimidin-6(5H)-yl)-2,4-difluorophenyl)-5-chloro-2-methoxypyridine-3-sulfonamide;
<22> N-(3-(2-amino-8-(difluoromethyl)-5-oxopirido[4,3-d]pyrimidin-6(5H)-yl)-2,4-difluorophenyl)-5-chloro-2-methoxypyridine-3-sulfonamide;
<23> N-(3-(2-amino-8-hydroxymethyl)-5-oxopirido[4,3-d]pyrimidin-6(5H)-2,4-difluorophenyl)-5-chloro-2-methoxypyridine-3-sulfonamide;
<24> N-(3-(2-amino-5-oxopirido[4,3-d]pyrimidin-6(5H)-yl)-2,4-difluorophenyl)-2,5-dichlorobenzenesulfonamide;
<25> N-(3-(2-amino-5-oxopirido[4,3-d]pyrimidin-6(5H)-yl)-2,4-difluorophenyl)-5-bromo-2-methoxypyridine-3-sulfonamide;
<2 6> N- (3- (2-amino-5-oxopirido[4,3-d]pyrimidin-6(5H)-yl)-2,4-difluorophenyl)-5-chloro-2-methoxypyridine-3-sulfonamide;
<27>N-(3-(2-amino-5-oxopirido[4,3-d]pyrimidin-6(5H)-yl)-2,4-difluorophenyl)-2,5-dichloropyridine-3-sulfonamide;
<28>N-(3-(2-amino-5-oxopirido[4,3-d]pyrimidin-6(5H)-yl)-2,4-difluorophenyl)-5-cyano-2-methoxypyridine-3-sulfonamide;
<29> N-(3-(2-amino-5-oxopirido[4,3-d]pyrimidin-6(5H)-yl)-2,4-difluorophenyl)naphthalene-1-sulfonamide;
<30> N-(3-(2-amino-5-oxopirido[4,3-d]pyrimidin-6(5H)-yl)-2,4-difluorophenyl)-5-chloro-1,3-dimethyl-1H-pyrazole-4-sulfonamide;
<31> N- (3- (2-amino-5-oxopirido[4,3-d]pyrimidin-6(5H)-yl)-2,4-difluorophenyl)-5-chlorothiophene-2-sulfonamide;
<32> 5-chloro-N-(2,4-difluoro-3-(2-(methylamino)-5-oxopirido[4,3-d]pyrimidin-6(5H)-yl)phenyl)-2-methoxypyridine-3-sulfonamide;
<33> N- (3- (2-amino-8-bromo-5-oxopirido[4,3-d]pyrimidin-6(5H)-yl)-2,4-difluorophenyl)-5-chloro-2-methoxypyridine-3-sulfonamide;
<34> N- (3- (2-amino-8-methyl-5-oxopyrimido[4, 5-d]pyridazine-6(5H)-yl)-2,4-difluorophenyl)-2,5-dichlorobenzenesulfonamide;
<35> N-(3-(2-amino-8-methyl-5-oxopyrimido[4,5-d]pyridazine-6(5H)-yl)-2,4-difluorophenyl)-1-methyl-3-(trifluoromethyl)-1H-pyrazole-4-sulfonamide;
<36> N- (3- (2-amino-8-methyl-5-oxopyrimido[4,5-d]pyridazine-6(5H)-yl)-2,4-difluorophenyl)-3-chloro-1,5-dimethyl- 1H-pyrazole-4-sulfonamide;
<37> N- (3- (2-amino-8-methyl-5-oxopyrimido[4,5-d]pyridazine-6(5H)-yl)-2,4-difluorophenyl)-2,5-dichloro-3-(hydroxymethyl)benzene sulfonamide;
<38> N-(3-(2-amino-8-methyl-5-oxopyrimido[4,5-d]pyridazine-6(5H)-yl)-2,4-difluorophenyl)-5-chloro-2-methoxypyridine-3-sulfonamide;
<39> N-(3-(7-amino-1-methyl-4-oxo-1,4-dihydropyrimido[4,5-d]pyrimidin-3(2H)-yl)-2,4-difluorophenyl)-2,5-dichlorobenzenesulfonamide;
<40> N-(3-(7-amino-1-methyl-4-oxo-1,4-dihydropyrimido[4,5-d]pyrimidin-3(2H)-yl)-2,4-difluorophenyl)-5-chloro-2-methoxypyridine-3-sulfonamide;
<41> N-(3-(7-amino-1-methyl-4-oxo-1,4-dihydropyrimido[4,5-d]pyrimidin-3(2H)-yl)-2,4-difluorophenyl)naphthalene-1-sulfonamide;
<42> N-(3-(7-amino-1-methyl-2,4-dioxo-1,4-dihydropyrimido[4,5-d]pyrimidin-3(2H)-yl)-2,4-difluorophenyl)-2,5-dichlorobenzenesulfonamide;
<43> N-(3-(7-amino-1-methyl-2,4-dioxo-1,4-dihydropyrimido[4,5-d]pyrimidin-3(2H)-yl)-2,4-difluorophenyl)naphthalene-1-sulfonamide;
<44> N-(3-(7-amino-1-methyl-2,4-dioxo-1,4-dihydropyrimido[4,5-d]pyrimidin-3(2H)-yl)-2,4-difluorophenyl)-5-chloro-2-methoxypyridine-3-sulfonamide;
<45> N-(3-(7-amino-1-methyl-2,4-dioxo-1,4-dihydropyrimido[4,5-d]pyrimidin-3(2H)-yl)-2,4-difluorophenyl)-5-chloro-2-methoxypyridine-3-sulfonamide;
<46> N-(3-(7-amino-1-methyl-2,4-dioxo-1,4-dihydropyrimido[4,5-d]pyrimidin-3(2H)-yl)-2,4-difluorophenyl)-5-bromo-2-methoxypyridine-3-sulfonamide;
<47> N-(3-(7-amino-1-methyl-2,4-dioxo-1,4-dihydropyrimido[4,5-d]pyrimidin-3(2H)-yl)-2,4-difluorophenyl)-5-cyano-2-methoxypyridine-3-sulfonamide;
<48> N-(3-(7-amino-1-methyl-2,4-dioxo-1,4-dihydropyrimido[4,5-d]pyrimidin-3(2H)-yl)-2,4-difluorophenyl)-1,3,5-trimethyl-1H-pyrazole-4-sulfonamide;
<49> N-(3-(7-amino-1-methyl-2,4-dioxo-1,2-dihydropyrimido[4,5-d]pyrimidin-3(4H)-yl)-2,4-difluorophenyl)-2-chloro-5-(trifluoromethyl)benzenesulfonamide;
<50> N-(3-(7-amino-1-methyl-2,4-dioxo-1,4-dihydropyrimido[4,5-d]pyrimidin-3(2H)-yl)-2,4-difluorophenyl)-5-chlorothiophene-2-sulfonamide;
<51> N-(3-(7-amino-1-methyl-2,4-dioxo-1,2-dihydropyrimido[4,5-d]pyrimidin-3(4H)-yl)-2,4-difluorophenyl)-5-bromo-2-methoxybenzenesulfonamide;
<52> N-(3-(7-amino-1-methyl-2,4-dioxo-1,4-dihydropyrimido[4,5-d]pyrimidin-3(2H)-yl)-2,4-difluorophenyl)-5-chloro-2-methylbenzenesulfonamide;
<53> N-(3-(7-amino-1-methyl-2,4-dioxo-1,4-dihydropyrimido[4,5-d]pyrimidin-3(2H)-yl)-2,4-difluorophenyl)-1-methyl-2-oxoindoline-5-sulfonamide;
<54> N-(3-(7-amino-1-methyl-2,4-dioxo-1,4-dihydropyrimido[4,5-d]pyrimidin-3(2H)-yl)-2,4-difluorophenyl)-2,5-difluorobenzenesulfonamide;
<55> N-(3-(7-amino-1-methyl-2,4-dioxo-1,2-dihydropyrimido[4,5-d]pyrimidin-3(4H)-yl)-2,4-difluorophenyl)-2,4-dichlorobenzenesulfonamide;
<56> N-(3-(7-amino-1-methyl-2,4-dioxo-1,4-dihydropyrimido[4,5-d]pyrimidin-3(2H)-yl)-2,4-difluorophenyl)-1-methyl-1H-pyrazole-4-sulfonamide;
<57> N-(3-(7-amino-1-methyl-2,4-dioxo-1,4-dihydropyrimido[4,5-d]pyrimidin-3(2H)-yl)-2,4-difluorophenyl)-2,5-dichloropyridine-3-sulfonamide;
<58> 5-chloro-N-(2,4-difluoro-3-(1-methyl-7-(methylamino)-2,4-dioxo-1,4-dihydropyrimido[4,5-d]pyrimidin-3(2H)-yl)phenyl)-2-methoxypyridine-3-sulfonamide;
<59> N-(3-(7-amino-1-cyclopropyl-4-oxo-1,4-dihydropyrimido[4,5-d]pyrimidin-3(2H)-yl)-2,4-difluorophenyl)-2,5-dichlorobenzenesulfonamide;
<60> N-(3-(7-amino-1-cyclopropyl-4-oxo-1,4-dihydropyrimido[4,5-d]pyrimidin-3(2H)-yl)-2,4-difluorophenyl)-5-chloro-2-methoxypyridine-3-sulfonamide;
<61> N-(3-(7-amino-1-cyclopropyl-4-oxo-1,4-dihydropyrimido[4,5-d]pyrimidin-3(2H)-yl)-2,4-difluorophenyl)naphthalene-1-sulfonamide;
<62> N-(3-(7-amino-1-cyclopropyl-4-oxo-1,4-dihydropyrimido[4,5-d]pyrimidin-3(2H)-yl)-2,4-difluorophenyl)-5-chloro-1,3-dimethyl-1H-pyrazole-4-sulfonamide;
<63> N-(3-(7-amino-1-cyclopropyl-2,4-dioxo-1,4-dihydropyrimido[4,5-d]pyrimidin-3(2H)-yl)-2,4-difluorophenyl)-2,5-dichlorobenzenesulfonamide;
<64> N-(3-(7-amino-1-cyclopropyl-2,4-dioxo-1,4-dihydropyrimido[4,5-d]pyrimidin-3(2H)-yl)-2,4-difluorophenyl)naphthalene-1-sulfonamide;
<65> N-(3-(7-amino-1-cyclopropyl-2,4-dioxo-1,4-dihydropyrimido[4,5-d]pyrimidin-3(2H)-yl)-2,4-difluorophenyl)-5-chloro-2-methoxypyridine-3-sulfonamide; and
<66> N-(3-(7-amino-1-cyclopropyl-2,4-dioxo-1,4-dihydropyrimido[4,5-d]pyrimidin-3(2H)-yl)-2,4-difluorophenyl)-5-chloro-1,3-dimethyl-1H-pyrazole-4-sulfonamide.

In one embodiment of the present invention, the present invention provides a preparation method of a compound represented by formula 1, comprising a step of reacting a compound represented by formula 2 with a compound represented by formula 3 to prepare a compound represented by formula 1, as shown in reaction formula 1 below.

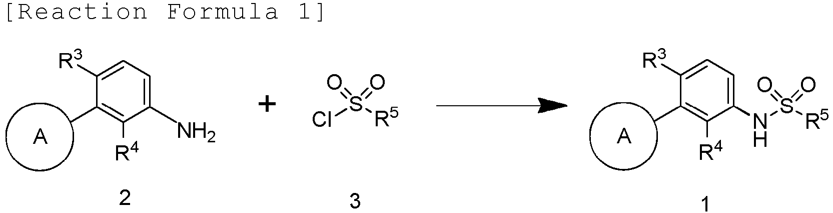

In reaction formula 1,
A, R¹, R², R³, R⁴, and R⁵ are as defined in formula 1 above.

The above coupling reaction can be carried out in an organic solvent such as dichloromethane, THF, ethyl acetate, or diethyl ether, in the presence of a base. At this time, a tertiary amine such as pyridine or trialkylamine can be used as the base, and the reaction can be carried out in the range of 0°C to 40°C, and can usually be performed at room temperature.

In another embodiment of the present invention, the present invention provides a preparation method of a compound represented by formula 1, comprising the following steps, as shown in reaction formula 2 below:
a step of reacting a compound represented by formula 2' with a compound represented by formula 3 to prepare a compound represented by formula 4; and
a step of reacting a compound represented by formula 4 with a compound represented by formula 5 to prepare a compound represented by formula 1.

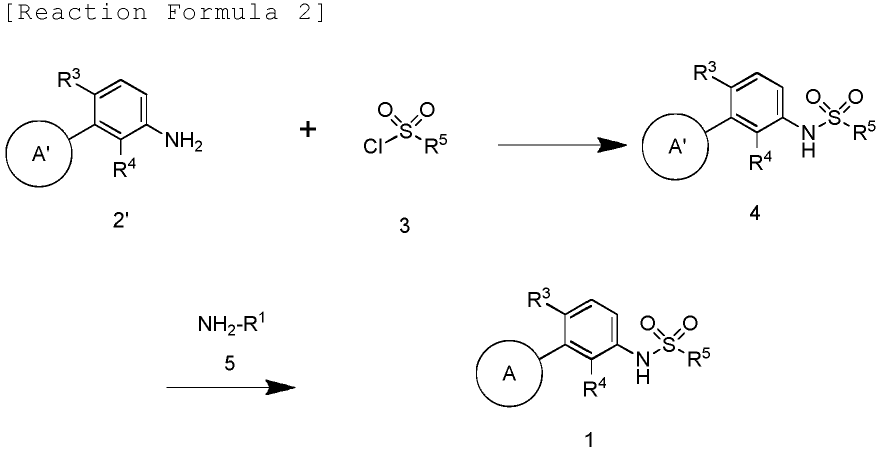

In reaction formula 2, A' is represented by a formula in which -SCH₃ is substituted for -NH-R¹ in the formula for A described above, and

A, R¹, R², R³, R⁴, and R⁵ are as defined in formula 1 above.

The first step in reaction formula 2 above can be carried out under the same conditions as the coupling reaction of reaction formula 1.

The second step is a reaction to substitute amine after oxidation of S in CH₃S, which is a reaction in which oxidation is performed using a peroxide such as mCPBA (meta-chloroperbezoic acid) in an organic solvent at a temperature range of -20°C to 20°C, and then amine is added in the organic solvent for substitution. At this time, when R¹ is hydrogen, formula 5 corresponds to ammonia (NH₃), and in this case, formula 5 can be supplied as ammonium hydroxide (NH₄OH) so that the substitution reaction is carried out in an organic solvent. The substitution reaction can be carried out in a polar organic solvent, for example, a lower alcohol such as ethanol or dioxane. It can be heated for the substitution reaction, and the heating can be performed in the range of 30°C to 100°C.

In one embodiment of the present invention, the present invention provides a pharmaceutical composition for the prevention or treatment of cancer, comprising a compound represented by formula 1 above, a stereoisomer thereof, a solvate thereof, a hydrate thereof, or a pharmaceutically acceptable salt thereof as an active ingredient.

The cancer can be leukemia, lymphoma, multiple myeloma, bladder cancer, breast cancer, colon cancer, kidney cancer, liver cancer, lung cancer, small cell lung cancer, esophageal cancer, gallbladder cancer, ovarian cancer, pancreatic cancer, stomach cancer, cervical cancer, thyroid cancer, prostate cancer, or skin cancer.

In one embodiment of the present invention, the present invention provides a pharmaceutical composition for the prevention or treatment of a disease caused by overactivation of kinases involved in ISR (integrated stress response), comprising a compound represented by formula 1 above, a stereoisomer thereof, a solvate thereof, a hydrate thereof, or a pharmaceutically acceptable salt thereof as an active ingredient.

The kinase involved in said ISR may be one or more selected from the group consisting of HRI (heme-regulated initiation factor-2a kinase), PKR (dsRNA-activated protein kinase), PERK (PKR-like endoplasmic reticulum kinase) and GCN2 (general control nonderepressible 2).

In the present invention, the term "comprising as an active ingredient" means containing in a dosage range that results in the prevention, amelioration, or treatment of cancer, wherein the dosage range may vary depending on the severity and formulation, and the number of applications may also vary depending on the age, weight, and constitution of the subject.

The pharmaceutical composition of the present invention is administered in a pharmaceutically effective dose.

In the present invention, the term "pharmaceutically effective dose" means an amount sufficient to treat a disease with a reasonable benefit/risk ratio applicable to medical treatment or improvement. The effective dose level depends on the factors including subject type and severity, age, gender, drug activity, sensitivity to drug, time of administration, administration route and excretion rate, duration of treatment, concomitant drugs, and other factors well known in the medical field. For example, 0.001 mg/kg to 100 mg/kg, 0.01 mg/kg to 10 mg/kg, or 0.1 mg/kg to 1 mg/kg is included in the effective amount. The upper limit of the dose of the pharmaceutical composition of the present invention can be selected by those skilled in the art within an appropriate range.

The pharmaceutical composition of the present invention can include a pharmaceutically effective dose of the compound represented by formula 1 alone or together with one or more pharmaceutically acceptable carriers, excipients, or diluents.

The said pharmaceutically acceptable carriers, excipients, or diluents herein indicate materials acceptable physiologically and do not cause any allergic reaction or allergy like reaction such as gastrointestinal disorder and dizziness. The carriers, excipients and diluents are exemplified by lactose, dextrose, sucrose, sorbitol, mannitol, xylitol, erythritol, maltitol, starch, acacia rubber, alginate, gelatin, calcium phosphate, calcium silcate, cellulose, methyl cellulose, polyvinyl pyrrolidone, water, methylhydroxybenzoate, propylhydroxybenzoate, talc, magnesium stearate and mineral oil, but not always limited thereto. In addition, fillers, antiaggregations, lubricants, wetting agents, flavors, emulsifiers, and antiseptics, can also be included.

The compound represented by formula 1 or the pharmaceutically acceptable salt thereof can be administered in a variety of oral and parenteral formulations during clinical administration. Solid formulations for oral administration are tablets, pills, powders, granules and capsules. These solid formulations are prepared by mixing one or more compounds with one or more suitable excipients such as starch, calcium carbonate, sucrose or lactose, gelatin, etc. Except for the simple excipients, lubricants, for example magnesium stearate, talc, etc, can be used. Liquid formulations for oral administrations are suspensions, solutions, emulsions and syrups, and the above-mentioned formulations can contain various excipients such as wetting agents, sweeteners, aromatics and preservatives in addition to generally used simple diluents such as water and liquid paraffin. Formulations for parenteral administration are sterilized aqueous solutions, water-insoluble excipients, suspensions, and emulsions. Water insoluble excipients and suspensions can contain, in addition to the active compound or compounds, propylene glycol, polyethylene glycol, vegetable oil like olive oil, injectable ester like ethylolate, etc.

The pharmaceutical composition comprising the compound represented by formula 1 or the pharmaceutically acceptable salt thereof as an active ingredient can be administered by parenterally and the parenteral administration includes subcutaneous injection, intravenous injection, intramuscular injection, or intrathoracic injection.

At this time, to prepare the compound represented by formula 1 or the pharmaceutically acceptable salt thereof as a formulation for parenteral administration, the compound represented by formula 1 or the pharmaceutically acceptable salt thereof is mixed with a stabilizer or a buffering agent in water to produce a solution or suspension, which is then formulated as ampoules or vials. The composition herein can be sterilized and additionally contains preservatives, stabilizers, wettable powders or emulsifiers, salts and/or buffers for the regulation of osmotic pressure, and other therapeutically useful materials, and the composition can be formulated by the conventional mixing, granulating or coating method.

In the present invention, the term "prevention" means preventing the occurrence of cancer symptoms in advance by administering, ingesting or applying the pharmaceutical composition or health functional food of the present invention to a subject not suffering from cancer to suppress or block the symptoms of cancer.

In the present invention, the term "treatment" includes complete cure of cancer symptoms as well as partial cure, amelioration and alleviation of cancer symptoms as a result of administering the pharmaceutical composition of the present invention to a subject suffering from cancer.

In addition, the compound represented by formula 1 above can be used in the form of not only its pharmaceutically acceptable salt but also isomers, solvates, hydrates, and the like that can be prepared therefrom.

The term "isomer" refers to a compound or a salt thereof of the present invention having the same chemical formula or molecular formula, but structurally or sterically different. These isomers include structural isomers such as tautomers and stereoisomers. Such stereoisomers include enantiomers having an asymmetric carbon center, diastereomers, and geometric isomers (trans, cis). In the present invention, all stereoisomers of the compound represented by formula 1 above and mixtures thereof are also included in the scope of the present invention.

The term "hydrate" refers to a compound or a salt thereof of the present invention containing a stoichiometric or non-stoichiometric amount of water bound by a non-covalent intermolecular force. The hydrate of the compound represented by formula 1 of the present invention can contain a stoichiometric or non-stoichiometric amount of water bonded by a non-covalent intermolecular force. The hydrate can contain 1 equivalent or more of water, preferably 1 to 5 equivalents of water. The hydrate can be prepared by crystallizing the compound represented by formula 1, the isomer thereof, or the pharmaceutically acceptable salt thereof from water or the solvent containing water.

The term "solvate" refers to a compound or a salt thereof of the present invention containing a stoichiometric or non-stoichiometric amount of solvent bound by a non-covalent intermolecular force. Preferred solvents therefor include those that are volatile, non-toxic, and/or suitable for administration to human.

The compound represented by formula 1 of the present invention can be used as a form of a pharmaceutically acceptable salt, in which the salt is preferably acid addition salt formed by pharmaceutically acceptable free acids. The acid addition salt herein can be obtained from inorganic acids such as hydrochloric acid, nitric acid, phosphoric acid, sulfuric acid, hydrobromic acid, hydroiodic acid, nitrous acid, and phosphorous acid; non-toxic organic acids such as aliphatic mono/dicarboxylate, phenyl-substituted alkanoate, hydroxy alkanoate, alkandioate, aromatic acids, and aliphatic/aromatic sulfonic acids; or organic acids such as acetic acid, benzoic acid, citric acid, lactic acid, maleic acid, gluconic acid, methanesulfonic acid, 4-toluenesulfonic acid, tartaric acid, and fumaric acid. The pharmaceutically non-toxic salts are exemplified by sulfate, pyrosulfate, bisulfate, sulphite, bisulphite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, suberate, cabacate, fumarate, maliate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, hydroxybutylate, glycolate, malate, tartrate, methanesulfonate, propanesulfonate, naphthalene-1-sulfonate, naphthalene-2-sulfonate, and mandelate.

The acid addition salt according to the present invention can be prepared by the conventional method known to those in the art. For example, the derivative represented by formula 1 is dissolved in an organic solvent such as methanol, ethanol, acetone, methylenechloride, and acetonitrile, to which organic acid or inorganic acid is added to induce precipitation. Then, the precipitate is filtered and dried to give the salt. Or the solvent and the excessive acid are distillated under reduced pressure, and dried to give the salt. Or the precipitate is crystallized in an organic solvent to give the same.

In addition, the pharmaceutical composition for the prevention or treatment of cancer comprising a compound represented by formula 1 above, a stereoisomer thereof, a solvate thereof, a hydrate thereof, or a pharmaceutically acceptable salt thereof as an active ingredient can be administered as an individual treatment, or can be used in combination with other treatment agents in use, and the effect can be enhanced by combination administration.

In one embodiment of the present invention, the present invention provides a health functional food for the prevention or amelioration of cancer, comprising a compound represented by formula 1 above, a stereoisomer thereof, a solvate thereof, a hydrate thereof, or a pharmaceutically acceptable salt thereof as an active ingredient.

In the present invention, the term "amelioration" means reducing or alleviating cancer symptoms by administering, ingesting, or applying the pharmaceutical composition or food composition of the present invention to a subject suffering from cancer.

The term "health functional food" means a food product manufactured and processed using raw materials or ingredients that have functional properties useful to the human body in accordance with the Health Functional Foods Act. The term "functional" food refers to a food that is consumed for the purpose of regulating nutrients for the structure and function of the human body or obtaining beneficial effects for health purposes such as physiological effects. The health functional food or health supplement of the present invention obtained in this way is very useful because it can be consumed on a daily basis.

There are no specific restrictions on the types of food, and all health functional foods in the ordinary sense are included.

The matters mentioned in the pharmaceutical composition and health functional food of the present invention are applied equally unless they are contradictory to each other.

In one embodiment of the present invention, the present invention provides a method for preventing or treating cancer comprising a step of administering a compound represented by formula 1 disclosed in the present specification, a stereoisomer thereof, a solvate thereof, a hydrate thereof, or a pharmaceutically acceptable salt thereof to a subject in need thereof.

In one embodiment of the present invention, the present invention provides a use of a compound represented by formula 1 disclosed in the present specification, a stereoisomer thereof, a solvate thereof, a hydrate thereof, or a pharmaceutically acceptable salt thereof for the production of a medicament for the prevention or treatment of cancer.

In the above method or use, the detailed description of the pharmaceutical composition described above may be applied.

Hereinafter, the present invention will be described in detail by the following preparative examples, examples, and experimental examples.

However, the following preparative examples, examples, and experimental examples are only for illustrating the present invention, and the contents of the present invention are not limited thereto.

### <Preparative Example>

The compounds of preparative examples 1 and 2 were prepared as shown in the scheme below.

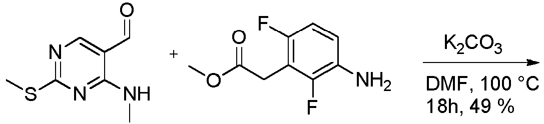

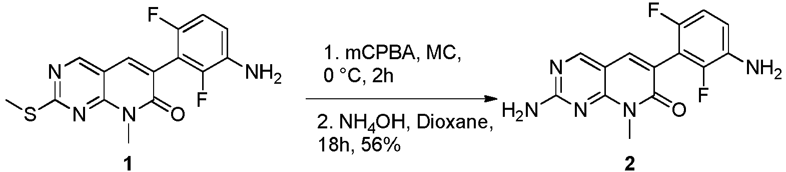

### <Preparative Example 1> 6-(3-amino-2,6-difluorophenyl)-8-methyl-2-(methylthio)pyrido[2,3-d]pyrimidin-7(8H)-one

4-(Methylamino)-2-(methylthio)pyrimidin-5-carbaldehyde (5.46 mmol, 1 g), methyl-2-(3-amino-2,6-difluorophenyl)acetate (6.55 mmol, 1.31g), and potassium carbonate (13.67 mmol, 2.26 g) were dissolved in 13 mL of DMF and reacted at 100°C for 18 hours. The reaction was terminated by adding aq. NH₄Cl and the solvent was removed by extraction with ethylacetate. The target compound was obtained (900 mg, yield: 49%) as a white solid by separation using a silica column (ethylacetate, hexane). ¹H NMR (400 MHz, DMSO-d₆) δ 9.72 (s, 1H), 8.57 (s, 1H), 8.31 (s, 1H), 6.78 - 6.59 (m, 2H), 3.74 (s, 3H), 3.61 (s, 2H), 3.14 (d, J = 5.0 Hz, 3H).

### <Preparative Example 2> 2-amino-6-(3-amino-2,6-difluorophenyl)-8-methylpyrido[2,3-d]pyrimidin-7(8H)-one

After adjusting the temperature of 6-(3-amino-2,6-difluorophenyl)-8-methyl-2-(methylthio)pyrido[2,3-d]pyrimidin-7(8H)-one(0.8 mmol, 290 mg) dissolved in 5 mL of DCM to 0°C, mCPBA (2.3 mmol, 410 mg) was slowly added thereto and reacted for 2.5 hours at room temperature. After confirming the completion of the reaction by TLC, the reaction was terminated with water, and the organic layer was separated to remove the solvent. The mixture was dissolved in 5 mL of dioxane, the reaction vessel was adjusted to 0°C, ammonium hydroxide (7.9 mmol, 0.3 mL) was added, sealed and allowed to react for 18 hours. Ethyl acetate was added to the reactant, the organic solvent was removed by separating from water, and separation was performed using a silica column (ethylacetate, hexane). As a result, the target compound was obtained (200 mg, yield: 83%) as a white solid.

¹H NMR (400 MHz, DMSO-d₆) δ 8.64 (s, 1H), 7.83 (s, 1H), 7.42 (s, 2H), 7.03 - 6.64 (m, 2H), 5.02 (s, 2H), 3.55 (s, 3H). m/z calculated for C₁₄H₁₁F₂N₅O⁺[M + H] : 303.2 Found: 305.1.

### <Preparative Example 3> 6-(3-amino-2,6-difluorophenyl)-8-methyl-2-(methylthio)pyrido[4,3-d]pyrimidin-5(6H)-one

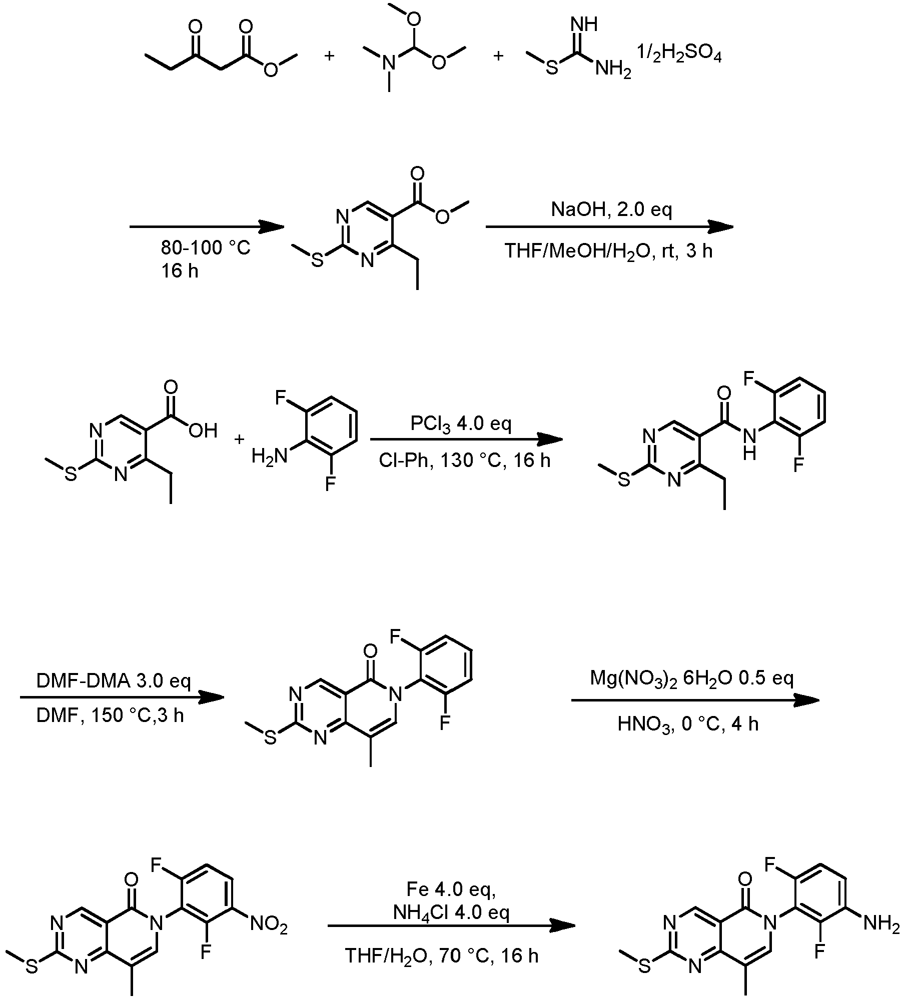

### Step 1: Synthesis of methyl-4-ethyl-2-(methylthio)pyrimidin-5-carboxylate

1,1-Dimethoxy-N,N-dimethylmethanamine (5.72 mL, 43.1 mmol) and methyl-3-oxopentanoate (4.67 g, 35.9 mmol) were added to a 500 mL round-bottom flask and reacted at 80°C for 10 minutes. Methyl carbamidothioate sulfate (5.0 g, 35.9 mmol) was added thereto, followed by reaction at 110°C for 16 hours. The reactant was cooled to room temperature, dissolved in EtOAc (80 mL) and transferred to a separatory funnel. The organic layer was washed with sat.NaHCO₃ (aq.) (1 x 80 mL)), water (1 x 80 mL), and brine (1 x 80 mL), dried over MgSO₄, filtered, and concentrated. The concentrate was purified by silica gel column chromatography (0-30% EtOAc) to give the target compound as a white solid (4.19 g, 52%).

¹H NMR (300 MHz, CDCl₃-*d*) δ 8.92 (s, 1H), 4.38 (q, J = 7.1, 2H), 3.14 (q, J = 7.5, 2H), 2.60 (s, 3H), 1.40 (t, J = 7.1, 3H), 1.29 (t, J = 7.5, 3H).

### Step 2: Synthesis of 4-ethyl-2-(methylthio)pyrimidin-5-carboxylic acid

In a 500 mL round-bottom flask, methyl-4-ethyl-2-(methylthio)pyrimidin-5-carboxylate (4.75 g, 22.38 mmol) was dissolved in THF/MeOH/H₂O 1:1:1 (54 mL), NaOH (2.69 g, 67.1 mmol) was added, and the mixture was reacted at room temperature for 16 hours. The mixture was then concentrated under reduced pressure to remove THF and MeOH. The concentrate was acidified (pH=2) by adding 1 N HCl (aq.). The resulting solid was filtered to give the target compound as a white solid (4.21 g, 95%).

¹H NMR (400 MHz, DMSO-d₆) δ 8.89 (s, 1H), 3.07 (q, J = 7.4 Hz, 2H), 2.55 (s, 3H), 1.20 (t, J = 7.5 Hz, 3H).

### Step 3: Synthesis of N-(2,6-difluorophenyl)-4-ethyl-2-(methylthio)pyrimidin-5-carboxamide

In a 500 mL round-bottom flask, 4-ethyl-2-(methylthio)pyrimidin-5-carboxylic acid (2.00 g, 10.09 mmol) was dissolved in benzene (25 mL) in a sealed tube. 2,6-Difluoroaniline (1.63 mL, 15.13 mmol) and trichlorophophane (3.53 mL, 40.04 mmol) were added thereto, followed by reaction at 130°C for 16 hours. After quenching with Sat. NaHCO₃ (aq.) (40 mL), the reactant was dissolved in DCM (20 mL) and transferred to a separatory funnel. After extraction with DCM (3 x 40 mL), the mixture was dried over MgSO₄ and concentrated under reduced pressure. The concentrate was dissolved in DCM (MeOH) and solidified with hexane to give the target compound as a yellow solid (1.23 g, 40%).

¹H NMR (400 MHz, DMSO-d₆) δ 10.40 (s, 1H), 8.72 (s, 1H), 7.43 (t, J = 7.4 Hz, 1H), 7.24 (t, J = 8.2 Hz, 2H), 2.91 (q, J = 7.6 Hz, 2H), 2.58 (s, 3H), 1.23 (t, J = 7.4 Hz, 3H).

### Step 4: Synthesis of 6-(2,6-difluorophenyl)-8-methyl-2-(methylthio)pyrido[4,3-d]pyrimidin-5(6H)-one

In a 100 mL round-bottom flask, the N-(2,6-difluorophenyl)-4-ethyl-2-(methylthio)pyrimidin-5-carboxamide (0.698 g, 2.256 mmol) obtained in step 3 was dissolved in DMF (6 mL), and 1,1-dimethoxy-N,N-dimethylmethanamine (0.9 mL, 6.77 mmol) was added, followed by reaction at 150°C for 3 hours. The reactant was dissolved in DCM (15 mL) and transferred to a separatory funnel. The organic layer was washed with H₂O (3 x 10 mL) and brine (1 x 10 mL), dried over MgSO₄, and concentrated. The concentrate was purified by silica gel column chromatography (0-40% EtOAc) to give the target compound as a yellow solid (0.263 g, 37%).

¹H NMR (400 MHz, DMSO-d₆) δ 9.27 (s, 1H), 7.96 (s, 1H), 7.73 - 7.62 (m, 1H), 7.41 (t, J = 8.5 Hz, 2H), 2.66 (s, 3H), 2.23 (s, 3H).

### Step 5: Synthesis of 6-(2,6-difluoro-3-nitrophenyl)-8-methyl-2-(methylthio)pyrido[4,3-d]pyrimidin-5(6H)-one

6-(2,6-Difluorophenyl)-8-methyl-2-(methylthio)pyrido[4,3-d]pyrimidin-5(6H)-one (0.298 g, 0.933 mmol) was dissolved in H₂SO₄ (1.5 mL) for 1.5 hr. The mixture was cooled to 0°C and magnesium nitrate hexahydrate (0.083 g, 0.560 mmol) was added portion wise, followed by reaction for 16 hours. The reaction was terminated by adding ice. The reactant was dissolved in EtOAc (10 mL), transferred to a separatory funnel, and extracted with EtOAc (3 x 15 mL). The extract was dried over MgSO₄, concentrated under reduced pressure, and then proceeded to the next reaction as a yellow solid without further purification.

### Step 6: Synthesis of 6-(3-amino-2,6-difluorophenyl)-8-methyl-2-(methylthio)pyrido[4,3-d]pyrimidin-5(6H)-one

The reaction mixture of step 5 (0.272 g, 0.933 mmol), Fe powder (0.167 g, 2.99 mmol), and NH₄Cl (0.160 g, 2.99 mmol) were dissolved in THF/MeOH/H₂O 2:2:1 (1.5 mL), followed by reaction at 70°C for 16 hours. The reactant was filtered through Celite and concentrated under reduced pressure. The concentrate was dissolved in EtOAc (7 mL) and transferred to a separatory funnel. It was washed with H₂O (1 x 7 mL) and brine (1 x 7 mL), dried over MgSO₄, and concentrated under reduced pressure. The concentrate was purified by silica gel column chromatography (0-50% EtOAc) to give the target compound as a yellow solid (0.0508 g, 20%).

¹H NMR (400 MHz, DMSO-d₆) δ 9.27 (s, 1H), 7.91 (s, 1H), 7.10 - 7.01 (m, 1H), 6.96 - 6.87 (m, 1H), 5.34 (s, 2H), 2.65 (s, 3H), 2.23 (s, 3H).

### <Preparative Example 4> Synthesis of 6-(3-amino-2,6-difluorophenyl)-8-(difluoromethyl)-2-(methylthio)pyrido[4,3-d]pyrimidin-5(6H)-one

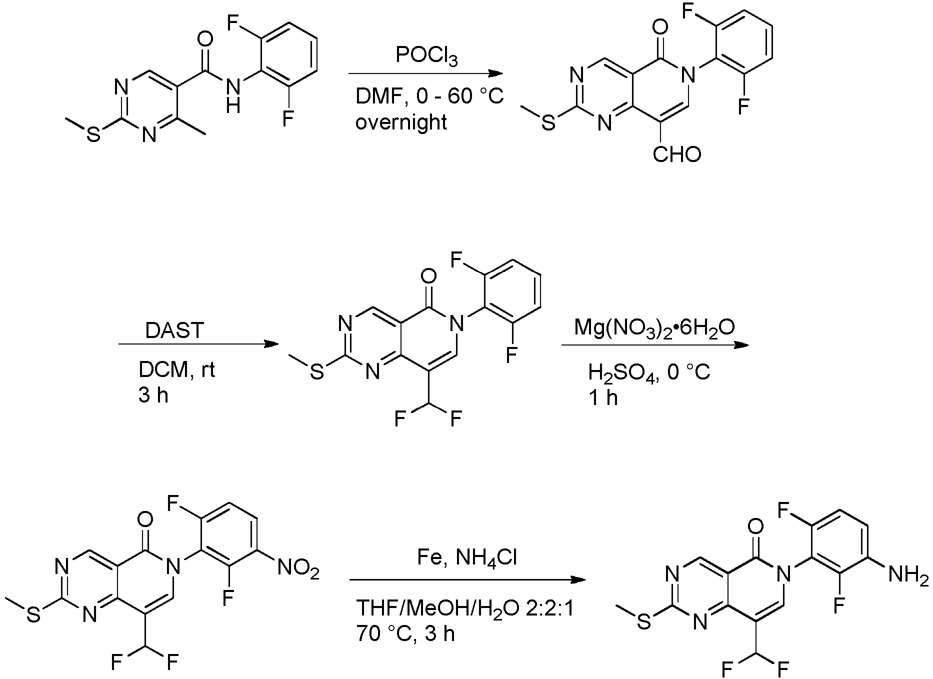

### Step 1: Synthesis of 6-(2,6-difluorophenyl)-2-(methylthio)-5-oxo-5,6-dihydropyrido[4,3-d]pyrimidin-8-carbaldehyde

RBF (50 mL) was vacuum-dried in an oven and purged with Ar (g). Distilled DMF (3 mL) was added to the mixture, and then POCl₃ (0.470 mL, 5.04 mmol) was added after cooling the mixture in an ice/water bath. After activating for 30 minutes while maintaining the temperature, N-(2,6-difluorophenyl)-4-methyl-2-(methylthio)pyrimidin-5-carboxamide (425 mg, 1.44 mmol) was dissolved in 2 mL of DMF and added dropwise to the mixture. The mixture was heated to 60°C and reacted overnight. After the reaction was terminated by adding ice, sat.NaHCO₃ (aq.) (10 mL) was slowly added. After stirring the reaction mixture at room temperature for 2 hours, the solid was filtered. The filtrate was transferred to a separatory funnel, to which EtOAc (100 mL) was added. The organic layer was washed with sat.NaHCO₃ (aq.) (100 mL) and brine (100 mL). The reactant was dried over MgSO₄ and concentrated under reduced pressure. The reaction mixture was purified by silica gel column chromatography (0~30% EtOAc/Hex) to give the target compound as a white solid (73 mg, 16%).

¹H NMR (400 MHz, CDCl₃) δ 10.77 (d, J = 1.6 Hz, 1H), 9.42 (d, J = 1.6 Hz, 1H), 8.25 (s, 1H), 7.59 - 7.51 (m, 1H), 7.18 (t, J = 8.7 Hz, 2H), 2.74 - 2.65 (m, 3H).

### Step 2: Synthesis of 8-(difluoromethyl)-6-(2,6-difluorophenyl)-2-(methylthio)pyrido[4,3-d]pyrimidin-5(6H)-one

After placing 6-(2,6-difluorophenyl)-2-(methylthio)-5-oxo-5,6-dihydropyrido[4,3-d]pyrimidin-8-carbaldehyde (70 mg, 0.210 mmol) in a 20 mL vial, DAST (0.111 mL, 0.840 mmol) was added. The mixture was reacted at room temperature for 3 hours and then the reaction was terminated by adding sat.NaHCO₃ (aq.) (5 mL). The reactant was transferred to a separatory funnel and extracted with DCM (15 mL x3). The extract was dried over MgSO₄ and concentrated under reduced pressure. The reaction mixture was purified by silica gel column chromatography (0~40% EtOAc/Hex) to give the target compound as a white solid (61 mg, 82%).

¹H NMR (400 MHz, CDCl₃) δ 9.40 (d, J = 1.8 Hz, 1H), 7.78 (s, 1H), 7.52 (ddd, J = 8.4, 6.4, 2.2 Hz, 1H), 7.25 (t, J = 55.0 Hz, 1H), 7.16 (td, J = 8.9, 1.6 Hz, 2H), 2.67 (d, *J* = 1.8 Hz, 3H).

### Step 3: Synthesis of 6-(2,6-difluoro-3-nitrophenyl)-8-(difluoromethyl)-2-(methylthio)pyrido[4,3-d]pyrimidin-5(6H)-one

After dissolving 8-(difluoromethyl)-6-(2,6-difluorophenyl)-2-(methylthio)pyrido[4,3-d]pyrimidin-5(6H)-one (77 mg, 0.217 mmol) in H₂SO₄ (2 mL), the temperature was lowered to 0°C. Mg(NO₃)2.6H₂O (32 mg, 0.127 mmol) was added to the above mixture and allowed to react for 3 hours. After the reaction was terminated by adding water (2 mL), the reactant was transferred to a separatory funnel. The reactant was completely dissolved in EtOAc (100 mL), and then washed with water (50 mL), sat.NaHCO₃ (aq.) (50 mL), and brine (50 mL). The reactant was dried over MgSO₄ and concentrated under reduced pressure. The reaction mixture was purified by silica gel column chromatography (0~30% EtOAc/Hex) to give the target compound as a pale yellow foam (39 mg, 45%).

¹H NMR (300 MHz, CDCl₃) δ 9.39 (s, 1H), 8.37 (ddd, J = 9.5, 8.1, 5.5 Hz, 1H), 7.73 (s, 1H), 7.32 (ddd, J = 9.7, 7.9, 2.1 Hz, 1H), 7.23 (t, J = 73.1 Hz, 1H), 2.67 (s, 3H).

### Step 4: Synthesis of 6-(3-amino-2,6-difluorophenyl)-8-(difluoromethyl)-2-(methylthio)pyrido[4,3-d]pyrimidin-5(6H)-one

After placing 6-(2,6-difluoro-3-nitrophenyl)-8-(difluoromethyl)-2-(methylthio)pyrido[4,3-d]pyrimidin-5(6H)-one (35 mg, 0.087 mmol) in a 20 mL vial, Fe (49 mg, 0.874 mmol) and NH₄Cl (47 mg, 0.874 mmol) were added. THF/MeOH/H₂O 1:1:1 (15 mL) was added to the above mixture and reacted at 70°C for 15 hours. After the temperature was allowed to drop to room temperature, the reaction mixture was filtered through celite. The filtrate was washed with EtOAc (100 mL), and the filtrate was transferred to a separatory funnel. The organic layer was washed with water (50 mL x1) and brine (50 mL x1), and then dried over MgSO₄.

### <Preparative Example 5> Synthesis of (6-(3-amino-2,6-difluorophenyl)-2-(methylthio)-5-oxo-5,6-dihydropyrido[4,3-d]pyrimidin-8-yl)methylacetate

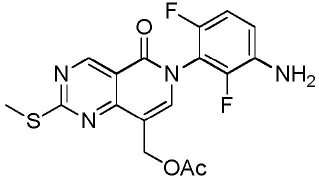

(6-(3-Amino-2,6-difluorophenyl)-2-(methylthio)-5-oxo-5,6-dihydropyrido[4,3-d]pyrimidin-8-yl)methylacetate was prepared in a similar manner to Preparative Example 4.

### <Preparative Example 6> Synthesis of 6-(3-amino-2,6-difluorophenyl)-2-(methylthio)pyrido[4,3-d]pyrimidin-5(6H)-one

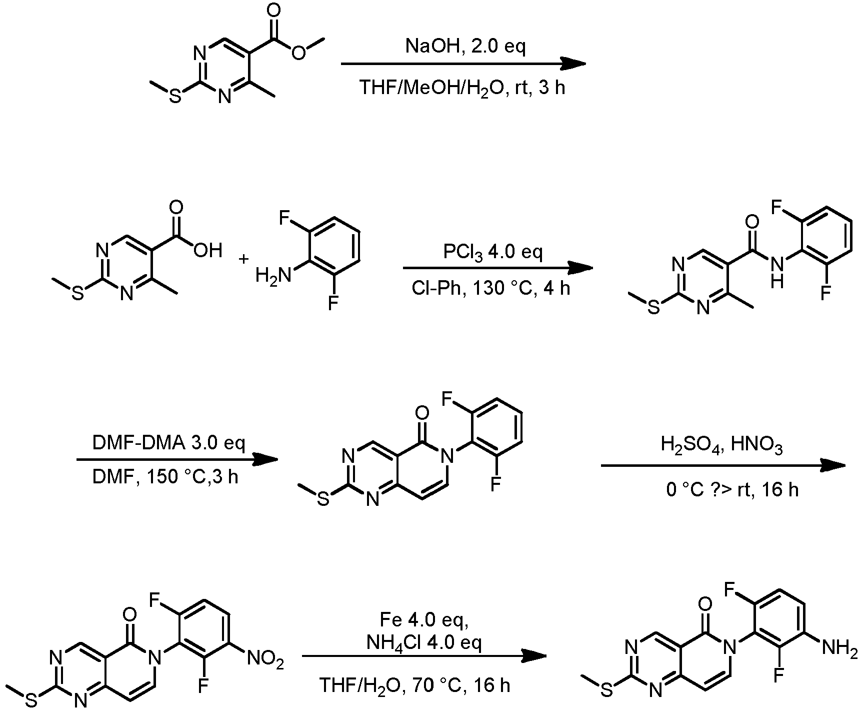

It was prepared in a similar manner to Preparative Example 3. Step 4 of the above scheme, i.e. the process for producing 6-(2,6-difluoro-3-nitrophenyl)-2-(methylthio)pyrido[4,3-d]pyrimidin-5(6H)-one, is as follows.

In a 50 mL round-bottom flask, add sulfuric acid (200 µL, 4.12 mmol) to the compound obtained in step 3 (110 mg, 0.34 mmol) and adjust the reaction vessel to 0°C. Nitric acid (30 µL, 0.66 mmol) was added thereto and reacted at room temperature for 16 hours. Ice water (1 mL) was added to the reactant to give the resulting solid and proceeded to the next reaction without further purification.

¹H NMR (400 MHz, DMSO-*d*₆) δ 9.30 (s, 1H), 8.52 (dt, J = 9.3, 4.5 Hz, 1H), 8.07 (d, J = 7.7 Hz, 1H), 7.72 (t, J = 9.3 Hz, 1H), 6.79 (d, J = 7.7 Hz, 1H), 2.64 (s, 3H).

### <Preparative Example 7> Synthesis of 6-(3-amino-2,6-difluorophenyl)-8-methyl-2-(methylthio)pyrimido[4,5-d]pyridazine-5(6H)-one

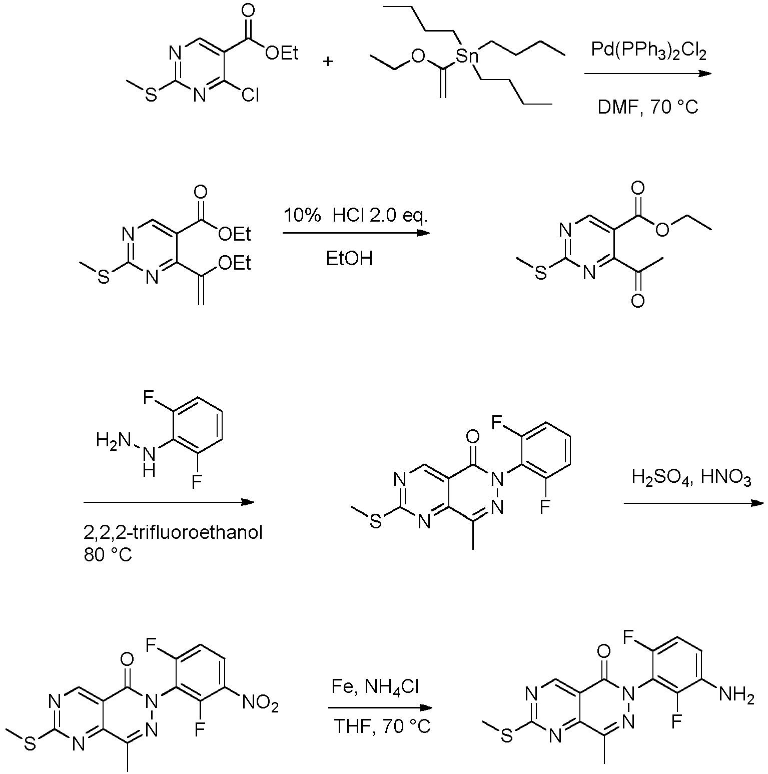

### Step 1: Synthesis of ethyl-4-(1-ethoxyvinyl)-2-(methylthio)pyrimidin-5-carboxylate

In a 100 mL round-bottom flask, 4-chloro-2-(methylthio)pyrimidin-5-carboxylate (1 g, 4.3 mmol), tributyl(1-ethoxyvinyl)stannane (1.8 g, 5.1 mmol), and bis (triphenylphosphine)palladium dichloride (60 mg, 8 mol%) were dissolved in 10 mL of N,N-dimethylformamide and reacted at 70°C for 1 hour. After the reaction mixture was cooled to room temperature, a solution of KF (1.5 g) dissolved in 3 mL of water was added to the reaction vessel and stirred for 18 hours. The reactant was passed through a celite filter to remove the solid, and extracted with ether. After evaporating the solvent under reduced pressure, the target compound was obtained by MPLC as a white product (830 mg, 72%).

¹H NMR (400 MHz, Chloroform-*d*) δ 8.69 (s, 1H), 5.22 (d, J = 2.5 Hz, 1H), 4.54 (d, J = 2.6 Hz, 1H), 4.38 (dq, J = 14.3, 7.1 Hz, 2H), 3.92 (q, J = 7.0 Hz, 2H), 2.63 (s, 3H), 1.47 - 1.31 (m, 6H).

### Step 2: Synthesis of ethyl-4-acetyl-2-(methylthio)pyrimidin-5-carboxylate

In a 100 mL round-bottom flask, ethyl-4-(1-ethoxyvinyl)-2-(methylthio)pyrimidin-5-carboxylate (830 mg, 3.0 mmol) was dissolved in 8 mL of ethanol, 10% HCl solution (1.8 mL, 6 mmol) was added thereto, and the mixture was reacted at 50°C for 18 hours. After confirming by TLC that the reaction was terminated, the solvent was removed under reduced pressure. After dissolving the reactant in EtOAc (7 mL), wash with water (1 × 7 mL) and collect the organic layer. Proceed to the next reaction without purification process. The target compound was obtained as a white solid (661 mg, 89%).

¹H NMR (400 MHz, Chloroform-*d*) δ 8.99 (s, 1H), 4.39 (q, J = 7.1 Hz, 2H), 2.63 (d, J = 1.8 Hz, 6H), 1.38 (t, J = 7.1 Hz, 3H).

### Step 3: Synthesis of 6-(2,6-difluorophenyl)-8-methyl-2-(methylthio)pyrimido[4,5-d]pyridazine-5(6H)-one

In a 100 mL round-bottom flask, 2,6-(difluorophenyl)hydrazine (300 mg, 2.0 mmol) and ethyl-4-(1-ethoxyvinyl)-2-(methylthio)pyrimidin-5-carboxylate (500 mg, 2.0 mmol) were dissolved in 10 mL of 2,2,2-trifluoroethanol and reacted at 80°C for 18 hours. The reaction was terminated by adding water, and the target compound was obtained as a yellow solid (250 mg, 38%).

¹H NMR (300 MHz, DMSO-*d*₆) δ 9.48 (s, 1H), 7.68 (tt, J = 8.5, 6.4 Hz, 1H), 7.40 (t, J = 8.3 Hz, 2H), 2.70 (s, 3H), 2.56 (s, 3H).

### Step 4: Synthesis of 6-(2,6-difluoro-3-nitrophenyl)-8-methyl-2-(methylthio)pyrimido[4,5-d]pyridazine-5(6H)-one

In a 50 mL round-bottom flask, add sulfuric acid (220 µL, 4.12 mmol) to the compound obtained in step 3 (110 mg, 0.34 mmol) and adjust the reaction vessel to 0°C. Nitric acid (30 µL, 0.68 mmol) was added thereto and reacted at room temperature for 18 hours. Add ice water (1 mL) to obtain the resulting solid. Then proceed similarly to step 6 of Preparative Example 3 to obtain the pyrimido compound. Then, 6-(3-amino-2,6-difluorophenyl)-8-methyl-2-(methylthio)pyrimido[4,5-d]pyridazine-5(6H)-one was obtained by proceeding similarly to step 6 in Preparative Example 3.

¹H NMR (400 MHz, DMSO-*d*₆) δ 9.50 (s, 1H), 8.59 - 8.46 (m, 1H), 7.70 (t, J = 9.3 Hz, 1H), 2.70 (s, 3H), 2.57 (s, 3H).

### <Preparative Example 8> Synthesis of 3-(3-amino-2,6-difluorophenyl)-1-methyl-7-(methylthio)-2,3-dihydropyrimido[4,5-d]pyrimidin-4(1H)-one

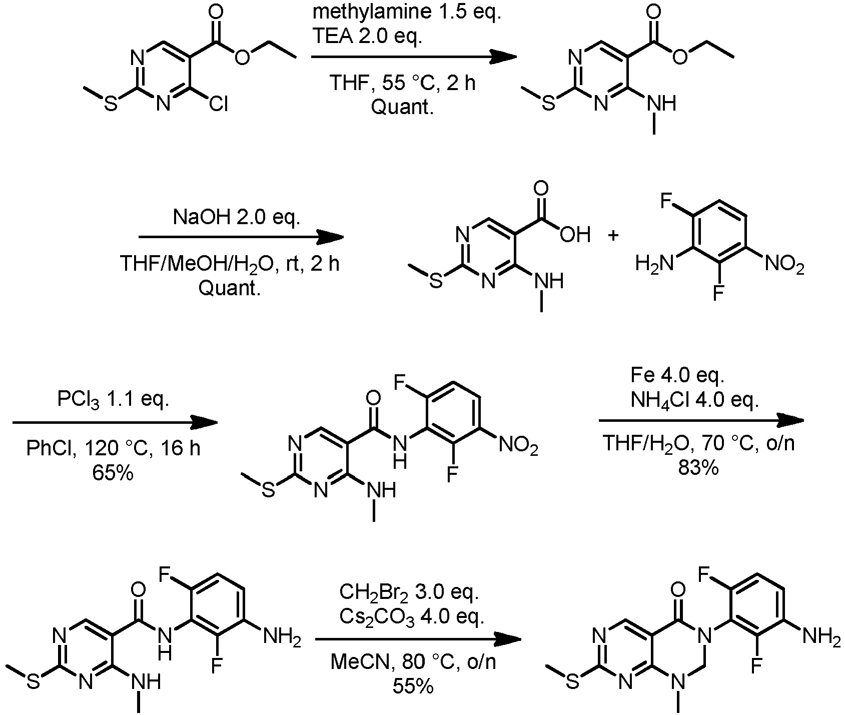

Ethyl-4-chloro-2-(methylthio)pyrimidin-5-carboxylate (1.0 g, 4.30 mmol) was dissolved in THF (22 mL), to which triethylamine (1.20 mL, 8.60 mmol) and methylamine (33 wt.% in ethanol, 0.78 mL, 6.45 mmol) were added sequentially at room temperature. Then, the temperature of the mixture was raised to 55°C and reacted for 2 hours. After confirming the completion of the reaction by TLC (EtOAc/HEX = 1/4), the temperature of the reaction was adjusted to room temperature. The mixture was diluted with H₂O and extracted with EtOAc. The extracted organic layer was dried over MgSO₄, filtered and concentrated under reduced pressure. The concentrate was purified by silica gel (EtOAc/HEX = 1/4, v/v) to give ethyl 4-(methylamino)-2-(methylthio)pyrimidin-5-carboxylate as a white solid (976 mg, yield: 99%).

¹H-NMR (400 MHz, CDCl₃) δ 8.60 (s, 1H), 8.16 (br, 1H), 4.31 (q, J = 7.2 Hz, 2H), 3.07 (d, J = 4.9 Hz, 3H), 2.54 (s, 3H), 1.36 (t, J = 7.1 Hz, 3H)

After that, similar reactions as steps 2, 3, and 6 of Preparative Example 3 were performed. For the synthesis of 3-(3-amino-2,6-difluorophenyl)-1-methyl-7-(methylthio)-2,3-dihydropyrimido[4,5-d]pyrimidin-4(1H)-one, N-(3-amino-2,6-difluorophenyl)-4-(methylamino)-2-(methylthio)pyrimidin-5-carboxamide (680 mg, 2.09 mmol) was dissolved in MeCN (10 mL), and then dibromomethane (0.44 mL, 6.27 mmol) and cesium carbonate (2.72 g, 8.36 mmol) were added sequentially at room temperature. Then, the temperature of the mixture was raised to 80°C and reacted for 15 hours. After confirming the completion of the reaction by TLC (EtOAc/HEX = 2/3), the reactant was adjusted to room temperature. The reactant was diluted with H₂O and extracted with EtOAc. The extracted organic layer was washed with brine, dried over MgSO₄, filtered, and concentrated under reduced pressure. The concentrate was purified with silica gel (EtOAc/HEX = 2/3, v/v) to give the target compound as a white solid (390 mg, yield: 55%).

### <Preparative Example 9> Synthesis of 3-(3-amino-2,6-difluorophenyl)-1-cyclopropyl-7 -(methylthio)-2,3-dihydropyrimido[4,5-di]pyrimidin-4(1H)-one

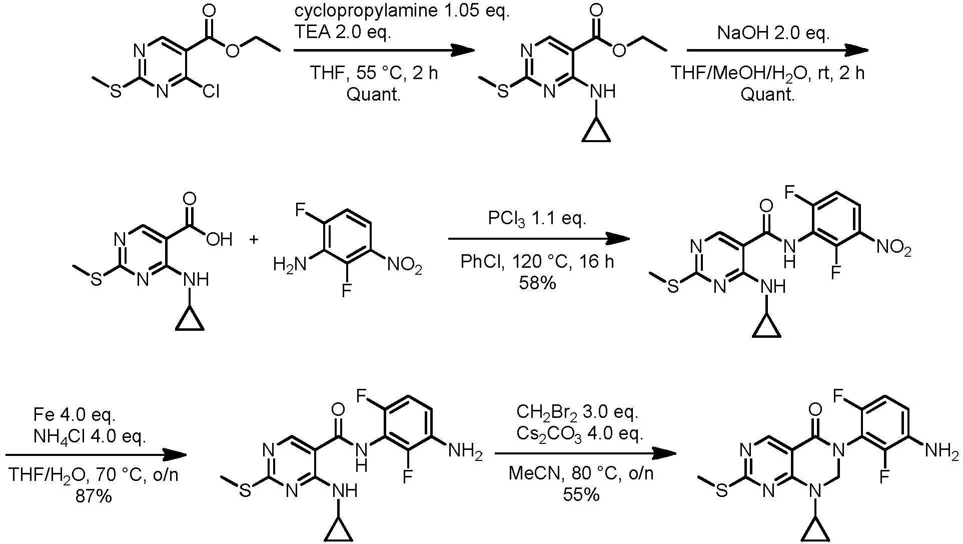

3-(3-Amino-2,6-difluorophenyl)-1-cyclopropyl-7-(methylthio)-2,3-dihydropyrimido[4,5-di]pyrimidin-4(1H)-one was prepared by a method similar to that described in Preparative Example 8.

### <Preparative Example 10> Synthesis of 3-(3-amino-2,6-difluorophenyl)-1-methyl-7-(methylthio)pyrimido[4,5-d]pyrimidin-2,4(1H,3H)-dione

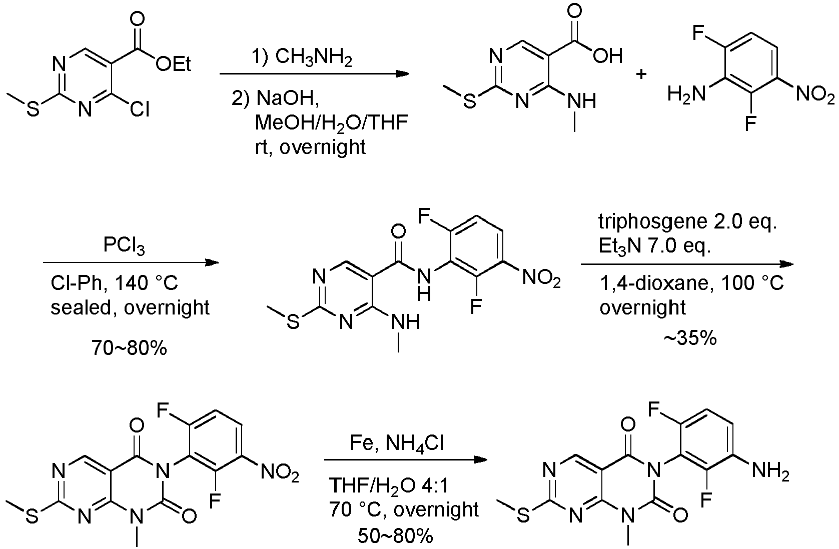

3-(3-Amino-2,6-difluorophenyl)-1-methyl-7-(methylthio)pyrimido[4,5-d]pyrimidin-2,4(1H,3H)-dione was prepared by a method similar to that described in Preparative Example 8.

¹H NMR (500 MHz, DMSO) δ 9.02 (s, 1H), 7.01 (t, J = 9.2, 9.2 Hz, 1H), 6.91 (t, J = 7.9, 7.9 Hz, 1H), 5.26 (s, 2H), 3.56 (s, 3H), 2.65 (s, 3H).

### <Preparative Example 11> Synthesis of 3-(3-amino-2,6-difluorophenyl)-1-cyclopropyl-7-(methylthio)pyrimido[4,5-d]pyrimidin-2,4(1H,3H)-dione

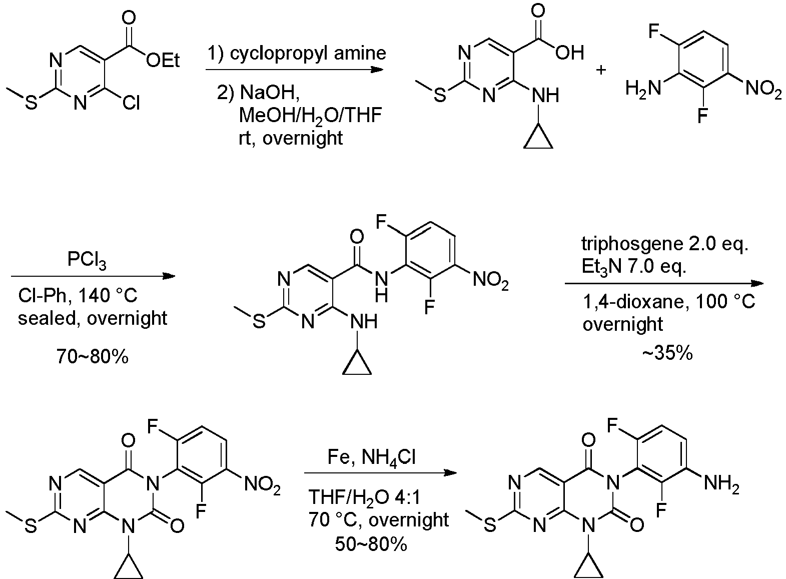

3-(3-Amino-2,6-difluorophenyl)-1-cyclopropyl-7-(methylthio)pyrimido[4,5-d]pyrimidin-2,4(1H,3H)-dione was prepared by a method similar to that described in Preparative Example 8.

### <Example 1> N-(3-(2-amino-8-methyl-7-oxo-7,8-dihydropyrido[2,3-d]pyrimidin-6-yl)-2,4-difluorophenyl)-1,3-dimethyl-5-(trifluoromethyl)-1H-pyrazole-4-sulfonamide

The 2-amino-6-(3-amino-2,6-difluorophenyl)-8-methylpyrido[2,3-d]pyrimidin-7(8H)-one (0.06 mmol, 20 mg) prepared in Preparative Example 1 was dissolved in 1 mL of dichloromethane, to which pyridine (0.65 mmol, 53 µL) and 1,3-dimethyl-5-(trifluoromethyl)-1H-pyrazole-4-sulfonyl chloride (0.09 mmol, 26 mg) were added, followed by reaction for 3 hours at room temperature. After evaporating the reaction solution, the reactant was purified through a silica column using ethylacetate and hexane to give the target compound as a light brown solid (15 mg, 43%).

¹H NMR (400 MHz, Chloroform-d) δ 8.50 (s, 1H), 7.64 (td, J = 9.0, 5.8 Hz, 1H), 7.53 (s, 1H), 7.03 (td, J = 8.7, 1.7 Hz, 1H), 5.46 (s, 3H), 3.88 (s, 3H), 3.73 (d, J = 2.9 Hz, 3H), 3.67 (s, 3H).

The compounds of Examples 2 to 19 were prepared as in Example 1.

**[Table 1]**

| Example | Compound Name | 1H NMR |
|---|---|---|
| 1 | N-(3-(2-amino-8-methyl-7-oxo-7,8-dihydropyrido[2,3-d]pyrimidin-6-yl)-2,4-difluorophenyl)-1,3-dimethyl-5-(trifluoromethyl)-1H-pyrazole-4-sulfonamide | ¹H NMR (400 MHz, Chloroform-d) δ 8.50 (s, |
| 2 | N-(3-(2-amino-8-methyl-7-oxo-7,8-dihydropyrido[2,3-d]pyrimidin-6-yl)-2,4-difluorophenyl)-5-chloro-1,3-dimethyl-1H-pyrazole-4-sulfonamide | 1H NMR (400 MHz, Chloroform-d) δ 8.52 (s, 1H), 7.62 (td, J = 8.9, 5.6 Hz, 1H), 7.57 (s, 1H), 7.17 (s, 1H), 6.99 (td, J = 8.8, 1.7 Hz, 1H), 5.51 (s, 2H), 3.82 (s, 3H), 3.70 (s, 3H), 2.33 (s, 3H). |
| 3 | N-(3-(2-amino-8-methyl-7-oxo-7,8-dihydropyrido[2,3-d]pyrimidin-6-yl)-2,4-difluorophenyl)-1,3,5-trimethyl-1H-pyrazole-4-sulfonamide | 1H NMR (400 MHz, Chloroform-d) δ 8.51 (s, 1H), 7.65 - 7.56 (m, 1H), 7.55 (d, J = 7.0 Hz, 1H), 7.05 - 6.92 (m, 1H), 6.76 (s, 1H), 5.47 (s, 2H), 3.73 (s, 3H), 3.69 (s, 3H), 2.32 (s, 2H), 2.25 (s, 3H). |
| 4 | N-(3-(2-amino-8-methyl-7-oxo-7,8-dihydropyrido[2,3-d]pyrimidin-6-yl)-2,4-difluorophenyl)-5-chloro-2-methoxypyridine-3-sulfonamide | 1H NMR (400 MHz, Chloroform-d) δ 8.51 (s, 1H), 8.28 (d, J = 2.5 Hz, 1H), 8.08 (d, J = 2.6 Hz, 1H), 7.65 - 7.58 (m, 1H), 7.56 (d, J = 7.6 Hz, 1H), 7.39 (s, 1H), 7.02 - 6.91 (m, 1H), 5.43 (s, 2H), 4.12 (s, 3H), 3.70 (s, 3H). |
| 5 | N-(3-(2-amino-8-methyl-7-oxo-7,8-dihydropyrido[2,3-d]pyrimidin-6-yl)-2,4-difluorophenyl)-3,5-dimethyl-1H-pyrazole-4-sulfonamide | 1H NMR (400 MHz, Chloroform-d) δ 8.54 (s, 1H), 8.22 (s, 1H), 7.49 - 7.38 (m, 1H), 7.06 (t, J = 9.0 Hz, 1H), 5.49 (d, J = 10.3 Hz, 2H), 5.33 (s, 1H), 3.79 (m, 3H), 3.14 (s, 3H), 3.06 (s, 3H). |
| 6 | N-(3-(2-amino-8-methyl-7-oxo-7,8-dihydropyrido[2,3-d]pyrimidin-6-yl)-2,4-difluorophenyl)-6-chloropyridine-3-sulfonamide | 1H NMR (400 MHz, Chloroform-d) δ 8.72 (d, J = 2.6 Hz, 1H), 8.52 (s, 1H), 7.76 - 7.58 (m, 2H), 7.50 (d, J = 12.2 Hz, 1H), 7.08 - 6.99 (m, 1H), 5.45 (s, 2H), 3.74 (s, 3H). |
| 7 | N-(3-(2-amino-8-methyl-7-oxo-7,8-dihydropyrido[2,3-d]pyrimidin-6-yl)-2,4-difluorophenyl)-1-methyl-1H-indole-4-sulfonamide | 1H NMR (400 MHz, Chloroform-d) δ 8.46 (s, 1H), 7.68 (d, J = 7.7 Hz, 1H), 7.57 (d, J = 8.3 Hz, 1H), 7.55 - 7.51 (m, 1H), 7.39 (s, 1H), 7.23 (d, J = 3.2 Hz, 1H), 6.93 - 6.86 (m, 1H), 6.84 (d, J = 3.4 Hz, 1H), 5.35 (s, 2H), 3.87 (s, 3H), 3.66 (s, 3H). |
| 8 | N-(3-(2-amino-8-methyl-7-oxo-7,8-dihydropyrido[2,3-d]pyrimidin-6-yl)-2,4-difluorophenyl)-1-(difluoromethyl)-3,5-dimethyl-1H-pyrazole-4-sulfonamide | 1H NMR (400 MHz, Chloroform-d) δ 8.50 (s, 1H), 7.50 (s, 1H), 7.44 (t, J = 7.9 Hz, 1H), 7.15 (s, 1H), 7.09 - 6.98 (m, 1H), 5.80 (s, 2H), 3.69 (s, 3H), 3.14 (s, 1H), 2.43 (s, 3H), 2.34 (s, 3H). |
| 9 | N-(3-(2-amino-8-methyl-7-oxo-7,8-dihydropyrido[2,3-d]pyrimidin-6-yl)-2,4-difluorophenyl)-3-chloro-1,5-dimethyl-1H-pyrazole-4-sulfonamide | 1H NMR (400 MHz, Chloroform-d) δ 8.51 (s, 1H), 8.22 (s, 1H) 7.61 (td, J = 8.7, 5.4 Hz, 1H), 6.99 (dd, J = 9.4, 7.8 Hz, 1H), 5.40 (d, J = 12.0 Hz, 2H), 3.77 (s, 3H), 3.69 (s, 3H), 2.28 (s, 3H), 2.07 |
| 10 | N-(3-(2-amino-8-methyl-7-oxo-7,8-dihydropyrido[2,3-d]pyrimidin-6-yl)-2,4-difluorophenyl)-3-(hydroxymethyl)-1,5-dimethyl-1H-pyrazole-4-sulfonamide | 1H NMR (400 MHz, Chloroform-d) δ 8.49 (s, 1H), 7.74 - 7.58 (m, 1H), 7.53 (s, 1H), 7.03 (td, J = 8.8, 1.7 Hz, 1H), 6.84 (s, 1H), 5.46 (s, 2H), 4.98 (d, J = 11.5 Hz, 1H), 4.67 (d, J = 11.5 Hz, 1H), 3.80 (s, 2H), 3.68 (s, 3H), 2.07 (d, J = 3.1 Hz, 3H). |
| 11 | N-(3-(2-amino-8-methyl-7-oxo-7,8-dihydropyrido[2,3-d]pyrimidin-6-yl)-2,4-difluorophenyl)-2-chloropyridine-3-sulfonamide | 1H NMR (400 MHz, Chloroform-d) δ 8.65 - 8.55 (m, 1H), 8.50 (s, 1H), 8.32 (dd, J = 7.8, 1.9 Hz, 1H), 7.63 - 7.54 (m, 1H), 7.54 (d, J = 11.0 Hz, 1H), 7.41 (dd, J = 7.8, 4.8 Hz, 1H), 6.95 (t, J = 8.5 Hz, 1H), 5.42 (s, 2H), 3.68 (s, 3H). |
| 12 | N-(3-(2-amino-8-methyl-7-oxo-7,8-dihydropyrido[2,3-d]pyrimidin-6-yl)-2,4-difluorophenyl)-2,5-difluorobenzenesulfon amide | 1H NMR (400 MHz, Chloroform-d) δ 8.52 (s, 1H), 7.62 (s, 1H), 7.40 (dddd, J = 13.8, 12.4, 6.9, 3.4 Hz, 2H), 7.25 (ddd, J = 9.0, 5.0, 2.5 Hz, 2H), 7.12 (ddd, J = 9.8, 8.6, 1.6 Hz, 1H), 5.57 (s, 2H), 3.70 (s, 3H). |
| 13 | N-(3-(2-amino-8-methyl-7-oxo-7,8-dihydropyrido[2,3-d]pyrimidin-6-yl)-2,4-difluorophenyl)-3, 5-dimethylisoxazole-4-sulfonamide | 1H NMR (400 MHz, Chloroform-d) δ 8.54 (s, 1H), 7.64 - 7.56 (m, 1H), 7.55 (d, J = 2.4 Hz, 1H), 7.08 - 6.99 (m, 1H), 6.73 (s, 1H), 5.42 (s, 2H), 3.71 (s, 3H), 2.48 (s, 3H), 2.34 (s, 3H). |
| 14 | N-(3-(2-amino-8-methyl-7-oxo-7,8-dihydropyrido[2,3-d]pyrimidin-6-yl)-2,4-difluorophenyl)-2,5-dichlorobenzenesulfon amide | 1H NMR (400 MHz, Chloroform-d) δ 8.52 (s, 1H), 8.03 (d, J = 2.0 Hz, 1H), 7.80 (s, 1H), 7.66 - 7.53 (m, 2H), 7.53 - 7.44 (m, 2H), 6.97 (td, J = 8.9, 1.8 Hz, 1H), 5.52 (s, 2H), 3.69 (s, 3H). |
| 15 | N-(3-(2-amino-8-methyl-7-oxo-7,8-dihydropyrido[2,3-d]pyrimidin-6-yl)-2,4-difluorophenyl)-6-chloroimidazo[2,1-b]thiazole-5-sulfonamide | 1H NMR (400 MHz, Chloroform-d) δ 8.53 (s, 1H), 7.80 (d, J = 4.5 Hz, 1H), 7.75 (s, 1H), 7.63 - 7.54 (m, 1H), 7.51 (s, 1H), 7.05 (d, J = 4.4 Hz, 1H), 6.98 (td, J = 8.8, 1.7 Hz, 1H), 5.51 (s, 3H), 3.68 (s, 3H). |
| 16 | N-(3-(2-amino-8-methyl-7-oxo-7,8-dihydropyrido[2,3-d]pyrimidin-6-yl)-2,4-difluorophenyl) naphthalene-1-sulfonamide | 1H NMR (400 MHz, Chloroform-d) δ 8.72 - 8.61 (m, 1H), 8.44 (s, 1H), 8.26 (dd, J = 7.4, 1.2 Hz, 1H), 8.09 (d, J = 8.2 Hz, 1H), 7.95 (dd, J = 7.8, 1.8 Hz, 1H), 7.62 (dqd, J = 8.2, 7.0, 1.5 Hz, 2H), 7.58 - 7.41 (m, 3H), 7.27 (s, 1H), 6.90 (td, J = 8.9, 1.7 Hz, 1H), 5.53 (s, 2H), 3.64 (s, 3H). |
| 17 | N-(3-(2-amino-8-methyl-7-oxo-7,8-dihydropyrido[2,3-d]pyrimidin-6-yl)-2,4-difluorophenyl)-1-methyl-3-(trifluoromethyl)-1H-pyrazole-4-sulfonamide | 1H NMR (400 MHz, Chloroform-d) δ 8.51 (s, 1H), 7.73 (s, 1H), 7.66 - 7.59 (m, 1H), 7.57 (d, J = 8.2 Hz, 1H), 7.01 (td, J = 8.8, 1.8 Hz, 1H), 5.50 (s, 2H), 3.99 (s, 3H), 3.69 (s, 3H). |
| 18 | N-(3-(2-amino-8-methyl-7-oxo-7,8-dihydropyrido[2,3-d]pyrimidin-6-yl)-2,4-difluorophenyl)-2-chloro-5-(trifluoromethyl)benz enesulfonamide | 1H NMR (400 MHz, Chloroform-d) δ 8.52 (s, 1H), 8.29 (d, J = 2.1 Hz, 1H), 8.18 (s, 1H), 7.76 (dd, J = 8.4, 2.2 Hz, 1H), 7.67 (d, J = 8.3 Hz, 1H), 7.63 - 7.54 (m, 2H), 6.97 (td, J = 8.9, 1.7 Hz, 1H), 5.59 (s, 2H), 3.68 (s, 3H). |
| 19 | N-(3-(2-amino-8-methyl-7-oxo-7,8-dihydropyrido[2,3-d]pyrimidin-6-yl)-2,4-difluorophenyl)-5-chlorothiophene-2-sulfonamide | 1H NMR (400 MHz, Chloroform-d) δ 8.52 (s, 1H), 7.65 - 7.60 (m, 1H), 7.55 (s, 1H), 7.34 (d, J = 4.1 Hz, 1H), 7.04 (dd, J = 8.8, 1.8 Hz, 2H), 6.92 (d, J = 4.0 Hz, 1H), 5.54 - 5.40 (m, 2H), 3.71 (s, 4H). |

### <Example 20> 2,5-dichloro-N-(2,4-difluoro-3-(8-methyl-2-(methylamino)-7-oxo-7,8-dihydropyrido[2,3-d]pyrimidin-6-yl)phenyl)benzenesulfonamide

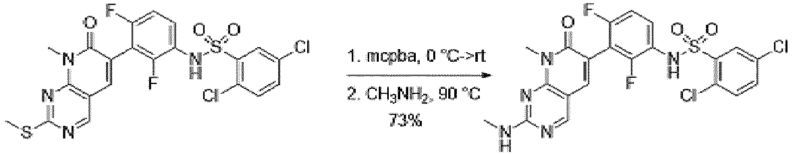

After proceeding with the reaction similarly to Example 1, 2,5-dichloro-N-(2,4-difluoro-3-(8-methyl-2-(methylthio)-7-oxo-7,8-dihydropyrido[2,3-d]pyrimidin-6-yl)phenyl)benzenesulfonamide (47 mg, 0.086 mmol) was dissolved in dichloromethane and mCPBA (16 mg, 1.1 mmol) was slowly added at 0°C. The reaction was terminated by adding water, the reactant was extracted with dichloromethane, and the organic solvent was evaporated. The mixture was dissolved in ethanol, to which 0.1 mL of methylamine was added, followed by reaction at 90°C for 3 hours. After evaporating the solvent, the reactant was purified through a silica column using ethylacetate/hexane to give the target compound (33 mg, 73%).

¹H NMR (500 MHz, Chloroform-d) δ 8.47 (s, 1H), 8.10 - 7.95 (m, 1H), 7.62 - 7.51 (m, 2H), 7.51 - 7.41 (m, 2H), 6.96 (td, J = 8.8, 1.7 Hz, 1H), 5.92 (s, 1H), 3.75 (s, 3H), 3.13 (d, J = 5.1 Hz, 3H).m/z calculated for: C₂₁H₁₅Cl₂F₂N₅O₃S⁺ [M + H]: 526.3 Found: 526.5

### <Example 21> N-(3-(2-amino-8-methyl-5-oxopirido[4,3-d]pyrimidin-6(5H)-yl)-2,4-difluorophenyl)-5-chloro-2-methoxypyridine-3- sulfinamide

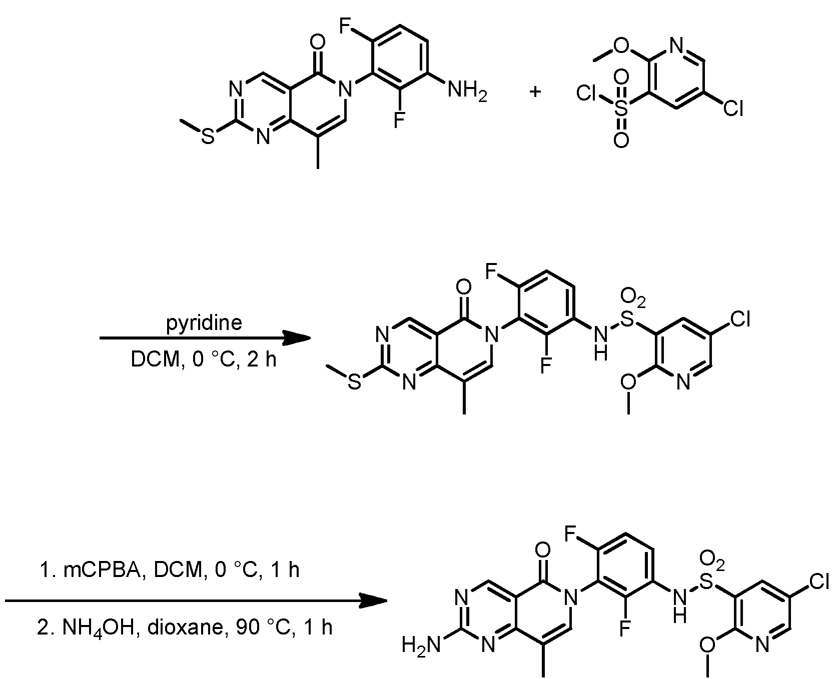

### Step 1: Synthesis of 5-chloro-N-(2,4-difluoro-3-(8-methyl-2-(methylthio)-5-oxopirido[4,3-d]pyrimidin-6(5H)-yl)phenyl)-2-methoxypyridine-3-sulfonamide

The 6-(3-amino-2,6-difluorophenyl)-8-methyl-2-(methylthio)pyrido[4,3-d]pyrimidin-5(6H)-one (0.0508 g, 0.152 mmol) prepared in Preparative Example 3 was dissolved in DCM (1 mL) and the temperature was lowered to 0°C, to which 5-chloro-2-methoxypyridine-3-dulfonyl chloride (0.044 g, 0.182 mmol) and pyridine (0.037 mL, 0.456 mmol) were added, followed by reaction at 0°C for 2 hours. The reaction was terminated by adding H₂O, and the reactant was dissolved in DCM (7 mL) and transferred to a separatory funnel. The reactant was extracted with DCM (3 × 7 mL), dried over MgSO₄, and concentrated under reduced pressure. The concentrate was purified by silica gel column chromatography (0-35% EtOAc) to give the target compound as a yellow oil (0.046 g, 56%).

¹H NMR (400 MHz, DMSO-*d*₆) δ 10.62 (s, 1H), 9.25 (s, 1H), 8.52 (s, 1H), 8.11 (s, 1H), 7.86 (s, 1H), 7.49 (d, J = 7.6 Hz, 1H), 7.37 (q, J = 9.1 Hz, 1H), 3.92 (s, 3H), 2.65 (s, 3H), 2.21 (s, 3H).

### Step 2: Synthesis of N-(3-(2-amino-8-methyl-5-oxopirido[4,3-d]pyrimidin-6(5H)-yl)-2,4-difluorophenyl)-5-chloro-2-methoxypyridine-3-sulfonamide

After dissolving 5-chloro-N-(2,4-difluoro-3-(8-methyl-2-(methylthio)-5-oxopirido[4,3-d]pyrimidin-6(5H)-yl)phenyl)-2-methoxypyridine-3-sulfonamide (0.046 g, 0.085 mmol) in DCM (1 mL), mCPBA (0.037 g, 0.213 mmol) was added thereto and reacted at 0°C for 1 hour. The reaction was terminated by adding H₂O (7 mL), and the reactant was dissolved in DCM (7 mL) and transferred to a separatory funnel. The reactant was extracted with DCM (3 × 7 mL), dried over MgSO_{4,} and concentrated under reduced pressure. The reaction mixture was dissolved in 1,4-dioxane (1 mL), to which NH₄OH (0.030 g, 0.850 mmol) was added, followed by reaction at 90°C for 1 hour. The reactant was dissolved in EtOAc (7 mL), transferred to a separatory funnel, and washed with H₂O (1 × 7 mL) and brine (1 × 7 mL). The reactant was dried over MgSO₄ and concentrated under reduced pressure. The concentrate was purified by silica gel column chromatography (0-5% MeOH) and concentrated under reduced pressure. The concentrate was dissolved in DCM (MeOH) and solidified with hexane to give the target compound as a white solid (5 mg, 12%).

¹H NMR (400 MHz, CDCl₃-*d*) δ 9.31 (s, 1H), 8.45 (s, 1H), 8.30 (d, J = 2.5 Hz, 1H), 8.11 (d, J = 2.6 Hz, 1H), 7.73 - 7.62 (m, 1H), 7.11 - 7.01 (m, 2H), 5.81 (s, 2H), 4.04 (s, 3H), 2.20 (s, 3H).

### <Example 22> N-(3-(2-amino-8-(difluoromethyl)-5-oxopirido[4,3-d]pyrimidin-6(5H)-yl)-2,4-difluorophenyl)-5-chloro-2-methoxypyridine-3-sulfonamide

N-(3-(2-amino-8-(difluoromethyl)-5-oxopirido[4,3-d]pyrimidin-6(5H)-yl)-2,4-difluorophenyl)-5-chloro-2-methoxypyridine-3-sulfonamide was prepared by a method similar to that described in Example 21.

¹H NMR (400 MHz, CDCl₃) δ 9.27 (s, 1H), 8.32 (d, J = 2.4 Hz, 1H), 8.10 (d, J = 2.5 Hz, 1H), 7.74 (s, 1H), 7.64 (s, 1H), 7.24 - 6.96 (m, 2H), 5.71 (s, 2H), 4.10 (d, J = 2.0 Hz, 3H).

### <Example 23> N-(3-(2-amino-8-hydroxymethyl)-5-oxopirido[4,3-d]pyrimidin-6(5H)-2,4-difluorophenyl)-5-chloro-2-methoxypyridine-3-sulfonamide

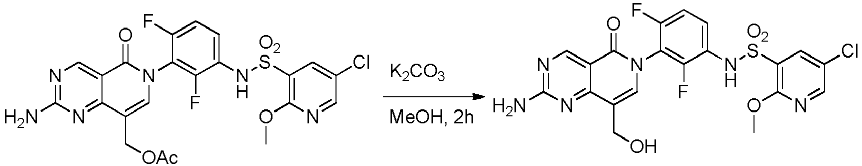

After preparing a compound protected with an acetyl group in a manner similar to the method described in Example 21, the target compound was prepared in the following manner.

The (2-amino-6-(3-((5-chloro-2-methoxypyridine)-3-sulfonamido)-2,6-difluorophenyl)-5-oxo-5,6-dihydropyrido[4,3-d]pyrimidin-8-yl)methylacetate (20 mg, 0.035 mmol) obtained in step 2 was dissolved in 1 mL of MeOH in a 10 mL vial, to which K₂CO₃ (10 mg, 0.07 mmol) was added, followed by reaction for 2 hours. After confirming the completion of the reaction by TLC, the pH of the reactant was adjusted to 6-7 using 1 N HCl, and the reactant was extracted with EtOAc. The extract was purified by MPLC to give the target compound as a white solid (5 mg, 27%).

¹H NMR (400 MHz, Chloroform-d) δ 9.28 (s, 1H), 8.31 (d, J = 2.5 Hz, 1H), 8.09 (d, J = 2.4 Hz, 1H), 7.72 (t, J = 7.7 Hz, 1H), 7.57 (d, J = 17.9 Hz, 1H), 7.17 (s, 1H), 7.09 (t, J = 9.0 Hz, 1H), 5.69 (s, 2H), 4.81 - 4.57 (m, 2H), 4.11 (d, J = 1.7 Hz, 3H).

The compounds of Examples 24 to 36 were prepared as in Example 21.

### <Example 37> N-(3-(2-amino-8-methyl-5-oxopyrimido[4,5-d]pyridazine-6(5H)-yl)-2,4-difluorophenyl)-2,5-dichloro-3-(hydroxymethyl)benzenesulfonamide

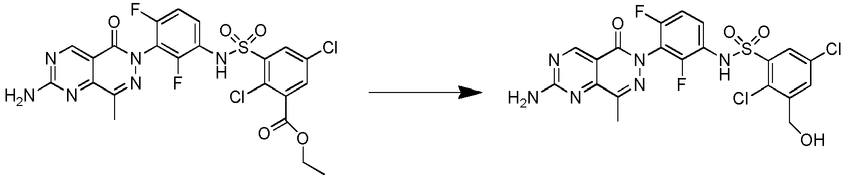

A compound substituted with ethylacetate (ethyl-3-(N-(3-(2-amino-8-methyl-5-oxopyrimido[4,5-d]pyridazine-6(5H)-yl)-2,4-difluorophenyl)sulfamoyl)-2,5-dichlorobezoic acid) was prepared in a manner similar to the method of Example 21. The compound (50 mg, 0.09 mmol) was dissolved in 1 mL of THF in a 50 mL round-bottom flask, to which 1 M LiAlH4 (171 µL, 0.17 mmol) was added at 0°C, followed by reaction for 30 minutes. The reaction was terminated by adding water, and the reactant was extracted with EA. The extract was purified by MPLC to obtain the target compound (15 mg, 32%).

The compounds of Examples 38 to 66 were prepared as in Example 21.

The compounds of Examples 24 to 66 are as follows.

**[Table 2]**

| Example | Compound Name | 1H NMR |
|---|---|---|
| 24 | N-(3-(2-amino-5-oxopirido[4,3-d]pyrimidin-6(5H)-yl)-2,4-difluorophenyl)-2,5-dichlorobenzenesulfon amide | 1H NMR (400 MHz, Chloroform-d) δ 9.28 (s, 1H), 8.19 (s, 1H), 8.03 (m, 1H), 7.71 - 7.65 (m, 1H), 7.53 - 7.48 (m, 3H), 7.16 (d, J = 7.7 Hz, 1H), 6.45 (d, J = 7.7 Hz, 1H), 7.27 (s, 1H), 7.13 - 7.09 (m, 1H), 5.90 (s, 2H). |
| 25 | N-(3-(2-amino-5-oxopirido[4,3-d]pyrimidin-6(5H)-yl)-2,4-difluorophenyl)-5-bromo-2-methoxypyridine-3-sulfonamide | 1H-NMR (400 MHz, CDCl₃) δ 9.27 (s, 1H), 8.44 (br, 1H), 8.37 (d, J = 2.5 Hz, 1H), 8.20 (d, J = 2.3 Hz, 1H), 7.68 (q, J = 8.4 Hz, 1H), 7.19 (m, 1H), 7.07 (m, 1H), 6.36 (d, J = 8.1 Hz, 1H), 5.79 (br, 2H), 4.01 (s, 3H). |
| 26 | N-(3-(2-amino-5-oxopirido[4,3-d]pyrimidin-6(5H)-yl)-2,4-difluorophenyl)-5-chloro-2-methoxypyridine-3-sulfonamide | H-NMR (300 MHz, CDCl₃) δ 9.27 (d, J = 0.7 Hz, 1H), 8.47 (br, 1H), 8.27 (d, J = 2.6 Hz, 1H), 8.08 (d, J = 2.6 Hz, 1H), 7.68 (ddd, J = 9.4, 8.4, 5.4 Hz, 1H), 7.19 (dd, J = 7.8, 1.4 Hz, 1H), 7.07 (td, J = 9.1, 2.0 Hz, 1H), 6.36 (dd, J = 7.8, 0.7 Hz, 1H), 5.86 (br, 2H), 4.01 (s, 3H). |
| 27 | N-(3-(2-amino-5-oxopirido[4,3-d]pyrimidin-6(5H)-yl)-2,4-difluorophenyl)-2,5-dichloropyridine-3-sulfonamide | ¹H-NMR (400 MHz, CDCl₃) δ 9.26 (s, 1H), 9.09 (br, 1H), 8.51 (d, J = 2.5 Hz, 1H), 8.30 (d, J = 2.5 Hz, 1H), 7.66 (m, 1H), 7.20 (d, J = 7.7 Hz, 1H), 7.10 (t, J = 8.8 Hz, 1H), 6.35 (d, J = 7.8 Hz, 1H), 5.87 (br, 2H). |
| 28 | N-(3-(2-amino-5-oxopirido[4,3-d]pyrimidin-6(5H)-yl)-2,4-difluorophenyl)-5-cyano-2-methoxypyridine-3-sulfonamide | ¹H-NMR (400 MHz, CDCl₃) δ 9.24 (s, 1H), 8.70 (d, J = 2.5 Hz, 1H), 7.72 (td, J = 8.8, 5.3 Hz, 1H), 7.52 (s, 1H), 7.23 - 7.14 (m, 2H), 6.41 (d, J = 7.7 Hz, 1H), 5.67 (s, 2H), 4.17 (s, 2H). |
| 29 | N-(3-(2-amino-5-oxopirido[4,3-d]pyrimidin-6(5H)-yl)-2,4-difluorophenyl)naphth alene-1-sulfonamide | 1H-NMR (300 MHz, CDCl₃) δ 9.19 (d, J = 0.6 Hz, 1H), 8.65 (m, 1H), 8.26 (dd, J = 7.4, 1.2 Hz, 1H), 8.09 (d, J = 8.2 Hz, 1H), 7.94 (m, 1H), 7.56 (m, 4H), 6.99 (td, J = 9.1, 2.1 Hz, 1H), 6.90 (d, J = 7.5 Hz, 1H), 6.27 (dd, J = 7.7, 0.7 Hz, 1H), 5.88 (br, 2H). |
| 30 | N-(3-(2-amino-5-oxopirido[4,3-d]pyrimidin-6(5H)-yl)-2,4-difluorophenyl)-5-chloro-1,3-dimethyl-1H-pyrazole-4-sulfonamide | 1H-NMR (300 MHz, CDCl3) δ 9.24 (d, J = 0.7 Hz, 1H), 8.09 (br, 1H), 7.70 (td, J = 8.9, 5.4 Hz, 1H), 7.20 (dd, J = 7.7, 1.3 Hz, 1H), 7.08 (td, J = 9.1, 2.0 Hz, 1H), 6.35 (dd, J = 7.8, 0.7 Hz, 1H), 5.84 (br, 2H), 3.79 (s, 3H), 2.31 (s, 3H). |
| 31 | N-(3-(2-amino-5-oxopirido[4,3-d]pyrimidin-6(5H)-yl)-2,4-difluorophenyl)-5-chlorothiophene-2-sulfonamide | 1H-NMR (300 MHz, CDCl3) δ 9.24 (s, 1H), 8.47 (br, 1H), 7.72 (td, J = 8.9, 5.4 Hz, 1H), 7.34 (d, J = 4.1 Hz, 1H), 7.20 (dd, J = 7.7, 1.3 Hz, 1H), 7.13 (td, J = 9.1, 2.0 Hz, 1H), 6.89 (d, J = 4.1 Hz, 1H), 6.35 (d, J = 7.7 Hz, 1H), 5.86 (br, 2H). |
| 32 | 5-chloro-N-(2,4-difluoro-3-(2-(methylamino)-5-oxopirido[4,3-d]pyrimidin-6(5H)-yl)phenyl)-2-methoxypyridine-3-sulfonamide | 1H-NMR (400 MHz, CDCl3) δ 9.17 (s, 1H), 8.28 (d, J = 2.6 Hz, 1H), 8.13 (br, 1H), 8.07 (d, J = 2.6 Hz, 1H), 7.66 (q, J = 7.9 Hz, 1H), 7.08 (m, 2H), 6.44 (d, J = 7.6 Hz, 1H), 5.99 (s, 1H), 4.05 (s, 3H), 3.12 (s, 3H). |
| 33 | N-(3-(2-amino-8-bromo-5-oxopirido[4,3-d]pyrimidin-6(5H)-yl)-2,4-difluorophenyl)-5-chloro-2-methoxypyridine-3-sulfonamide | 1H NMR (400 MHz, Chloroform-d) δ 8.74 (s, 1H), 8.33 (s, 2H), 7.56 (s, 1H), 7.43 (d, J = 8.3 Hz, 2H), 6.96 (s, 1H), 6.45 (s, 2H), 4.17 (s, 3H). |
| 34 | N-(3-(2-amino-8-methyl-5-oxopyrimido[4,5-d]pyridazine-6(5H)-yl)-2,4-difluorophenyl)-2,5-dichlorobenzenesulfon amide | 1H NMR (400 MHz, Chloroform-d) δ 9.36 (d, J = 1.4 Hz, 1H), 8.11 (d, J = 1.9 Hz, 1H), 7.54 - 7.48 (m, 2H), 7.48 - 7.42 (m, 1H), 7.06 (t, J = 9.1 Hz, 1H), 6.18 (s, 2H), 2.51 (d, J = 1.5 Hz, 3H). |
| 35 | N-(3-(2-amino-8-methyl-5-oxopyrimido[4,5-d]pyridazine-6(5H)-yl)-2,4-difluorophenyl)-1-methyl-3-(trifluoromethyl)-1H-pyrazole-4-sulfonamide | 1H NMR (400 MHz, Chloroform-d) δ 9.31 (s, 1H), 7.80 - 7.64 (m, 2H), 7.18 - 7.10 (m, 1H), 5.89 (s, 2H), 3.99 (s, 3H), 2.51 (s, 3H). |
| 36 | N-(3-(2-amino-8-methyl-5-oxopyrimido[4,5-d]pyridazine-6(5H)-yl)-2,4-difluorophenyl)-3-chloro-1,5-dimethyl-1H-pyrazole-4-sulfonamide | 1H NMR (400 MHz, Chloroform-d) δ 9.35 (s, 1H), 7.71 (td, J = 8.9, 5.3 Hz, 1H), 7.18 (s, 1H), 7.08 (td, J = 9.1, 1.9 Hz, 1H), 5.99 (s, 2H), 3.82 (s, 3H), 2.52 (s, 3H), 2.36 (s, 3H). |
| 37 | N-(3-(2-amino-8-methyl-5-oxopyrimido[4,5-d]pyridazine-6(5H)-yl)-2,4-difluorophenyl)-2,5-dichloro-3-(hydroxymethyl)benzen e sulfonamide | 1H NMR (300 MHz, Chloroform-d) δ 9.26 (s, 1H), 8.31 (d, J = 2.6 Hz, 1H), 8.11 (d, J = 2.6 Hz, 1H), 7.75 (s, 1H), 7.44 (t, J = 7.9 Hz, 1H), 7.39 (s, 1H), 7.09 (td, J = 9.0, 2.0 Hz, 1H), 5.93 (s, 3H), 5.21 (s, 3H), 4.10 (s, 3H). |
| 38 | N-(3-(2-amino-8-methyl-5-oxopyrimido[4,5-d]pyridazine-6(5H)-yl) -2, 4-difluorophenyl)-5-chloro-2-methoxypyridine-3-sulfonamide | 1H NMR (300 MHz, Chloroform-d) δ 9.26 (s, 1H), 8.31 (d, J = 2.6 Hz, 1H), 8.11 (d, J = 2.6 Hz, 1H), 7.71 (dd, J = 9.9, 4.5 Hz, 2H), 7.51 - 7.40 (m, 1H), 7.39 (s, 1H), 7.09 (td, J = 9.0, 2.0 Hz, 1H), 5.93 (s, 2H), 4.10 (s, 3H), 2.12 (s, 3H). |
| 39 | N-(3-(7-amino-1-methyl-4-oxo-1,4-dihydropyrimido[4,5-d]pyrimidin-3(2H)-yl)-2,4-difluorophenyl)-2,5-dichlorobenzenesulfon amide | 1H-NMR (300 MHz, CDCl3) δ 8.59 (s, 1H), 7.99 (t, J = 1.4 Hz, 1H), 7.49 (m, 3H), 6.97 (td, J = 9.2, 2.1 Hz, 1H), 5.54 (br, 2H), 4.85 (d, J = 9.2 Hz, 1H), 4.73 (d, J = 9.2 Hz, 1H), 3.08 (s, 3H). |
| 40 | N-(3-(7-amino-1-methyl-4-oxo-1,4-dihydropyrimido[4,5-d]pyrimidin-3(2H)-yl)-2,4-difluorophenyl)-5-chloro-2-methoxypyridine-3-sulfonamide | 1H-NMR (300 MHz, CDCl3) δ 8.60 (s, 1H), 8.27 (d, J = 2.6 Hz, 1H), 8.06 (d, J = 2.6 Hz, 1H), 7.53 (td, J = 8.9, 5.4 Hz, 1H), 6.96 (td, J = 9.2, 2.0 Hz, 1H), 5.61 (br, 2H), 4.87 (d, J = 9.4 Hz, 1H), 4.70 (d, J = 9.2 Hz, 1H), 4.07 (s, 3H), 3.08 (s, 3H). |
| 41 | N-(3-(7-amino-1-methyl-4-oxo-1,4-dihydropyrimido[4,5-d]pyrimidin-3(2H)-yl)-2,4-difluorophenyl)naphth alene-1-sulfonamide | 1H-NMR (300 MHz, CDCl3) δ 8.64 (m, 1H), 8.55 (s, 1H), 8.23 (dd, J = 7.4, 1.2 Hz, 1H), 8.07 (d, J = 8.2 Hz, 1H), 7.93 (m, 1H), 7.61 (m, 2H), 7.51 (dd, J = 8.2, 7.4 Hz, 1H), 7.40 (td, J = 8.9, 5.4 Hz, 1H), 6.88 (td, J = 9.2, 2.0 Hz, 1H), 5.42 (br, 2H), 4.61 (d, J = 9.3 Hz, 1H), 4.41 (d, J = 9.1 Hz, 1H), 3.00 (s, 3H). |
| 42 | N-(3-(7-amino-1-methyl-2,4-dioxo-1,4-dihydropyrimido[4,5-d]pyrimidin-3(2H)-yl)-2,4-difluorophenyl)-2,5-dichlorobenzenesulfon amide | 1H NMR (300 MHz, CDCl3 CDCl3-d) δ 8.94 (s, 1H), 8.03 (d, J = 2.2 Hz, 1H), 7.66 (td, J = 8.9, 5.3 Hz, 1H), 7.54 - 7.41 (m, 2H), 7.04 (t, J = 8.9 Hz, 1H), 5.81 (s, 2H), 3.57 (s, 3H). |
| 43 | N-(3-(7-amino-1-methyl-2,4-dioxo-1,4-dihydropyrimido[4,5-d] pyrimidin-3 (2H) - yl)-2,4-difluorophenyl)naphth alene-1-sulfonamide | 1H NMR (300 MHz, CDCl3-d) δ 8.87 (s, 1H), 8.67 (d, J = 8.6 Hz, 1H), 8.19 (dd, J = 7.4, 1.3 Hz, 1H), 8.08 (d, J = 8.2 Hz, 1H), 7.94 (d, J = 8.6 Hz, 1H), 7.73 - 7.45 (m, 4H), 7.01 - 6.89 (m, 1H), 5.68 (s, 2H), 3.52 (s, 3H). |
| 44 | N-(3-(7-amino-1-methyl-2,4-dioxo-1,4-dihydropyrimido[4,5-d]pyrimidin-3(2H)-yl)-2,4-difluorophenyl)-5-chloro-2-methoxypyridine-3-sulfonamide | 1H NMR (300 MHz, CDCl3-d ) δ 8.92 (s, 1H), 8.27 (d, J = 2.6 Hz, 1H), 8.07 (d, J = 2.5 Hz, 1H), 7.74 - 7.60 (m, 1H), 7.03 (td, J = 9.0, 2.1 Hz, 1H), 5.72 (s, 2H), 4.05 (s, 3H), 3.57 (s, 3H). |
| 45 | N-(3-(7-amino-1-methyl-2,4-dioxo-1,4-dihydropyrimido[4,5-d]pyrimidin-3(2H)-yl)-2,4-difluorophenyl)-5-chloro-2-methoxypyridine-3-sulfonamide | 1H NMR (400 MHz, CDCl3-d) δ 8.91 (s, 1H), 7.73 - 7.65 (m, 1H), 7.06 (t, J = 8.9 Hz, 1H), 6.84 (s, 1H), 5.67 (s, 3H), 3.79 (s, 3H), 3.57 (s, 2H), 2.33 (s, 3H). |
| 46 | N-(3-(7-amino-1-methyl-2,4-dioxo-1,4-dihydropyrimido[4,5-d] pyrimidin-3 (2H) - yl)-2,4-difluorophenyl)-5-bromo-2-methoxypyridine-3-sulfonamide | 1H NMR (300 MHz, CDCl3-d) δ 8.93 (s, 1H), 8.36 (d, J = 2.4 Hz, 1H), 7.67 (td, J = 9.1, 5.4 Hz, 2H), 7.03 (td, J = 9.0, 2.0 Hz, 1H), 5.72 (s, 2H), 4.03 (s, 3H), 3.57 (s, 3H). |
| 47 | N-(3-(7-amino-1-methyl-2,4-dioxo-1,4-dihydropyrimido[4,5-d] pyrimidin-3 (2H) - yl)-2,4-difluorophenyl)-5-cyano-2-methoxypyridine-3-sulfonamide | 1H NMR (400 MHz, CDCl3-d) δ 8.92 (d, J = 1.8 Hz, 1H), 8.63 (d, J = 2.4 Hz, 1H), 8.17 (d, J = 2.4 Hz, 1H), 7.72 (q, J = 8.1, 7.5 Hz, 1H), 7.38 (s, 1H), 7.14 (t, J = 8.9 Hz, 1H), 5.91 (s, 2H), 4.16 (t, J = 1.7 Hz, 3H), 3.58 (d, J = 1.8 Hz, 3H). |
| 48 | N-(3-(7-amino-1-methyl-2,4-dioxo-1,4-dihydropyrimido[4,5-d]pyrimidin-3(2H)-yl)-2,4-difluorophenyl)-1,3,5-trimethyl-1H-pyrazole-4-sulfonamide | 1H NMR (400 MHz, CDCl3-d) δ 8.91 (d, J = 1.6 Hz, 1H), 7.71 (d, J = 6.6 Hz, 1H), 7.14 - 7.05 (m, 1H), 6.74 (s, 1H), 5.75 (s, 2H), 3.71 (d, J = 1.5 Hz, 3H), 3.58 (d, J = 1.4 Hz, 3H), 2.38 (d, J = 1.5 Hz, 3H), 2.05 (d, J = 1.7 Hz, 3H). |
| 49 | N-(3-(7-amino-1-methyl-2,4-dioxo-1,2-dihydropyrimido[4,5-d]pyrimidin-3(4H)-yl)-2,4-difluorophenyl)-2-chloro-5-(trifluoromethyl)benz enesulfonamide | 1H NMR (400 MHz, CDCl3-d) δ 8.91 (d, J = 1.9 Hz, 1H), 8.28 (s, 1H), 7.76 (d, J = 8.4 Hz, 1H), 7.73 - 7.61 (m, 3H), 7.03 (t, J = 9.2 Hz, 1H), 5.73 (d, J = 60.0 Hz, 3H), 3.55 (t, J = 1.8 Hz, 3H). |
| 50 | N-(3-(7-amino-1-methyl-2,4-dioxo-1,4-dihydropyrimido[4,5-d]pyrimidin-3(2H)-yl)-2,4-difluorophenyl)-5-chlorothiophene-2-sulfonamide | 1H NMR (500 MHz, CDCl3-d) δ 8.91 (s, 1H), 7.75 - 7.67 (m, 1H), 7.30 (s, 1H), 7.24 (d, J = 4.1 Hz, 1H), 7.10 (t, J = 8.9 Hz, 1H), 6.88 (d, J = 4.1 Hz, 1H), 5.63 (s, 2H), 3.58 (s, 3H). |
| 51 | N-(3-(7-amino-1-methyl-2,4-dioxo-1,2-dihydropyrimido[4,5-d]pyrimidin-3(4H)-yl)-2,4-difluorophenyl)-5-bromo-2-methoxybenzenesulfonamide | 1H NMR (400 MHz, CDCl3-d) δ 8.92 (d, J = 1.8 Hz, 1H), 8.01 - 7.95 (m, 1H), 7.72 - 7.58 (m, 2H), 7.30 (s, 1H), 6.99 (t, J = 9.1 Hz, 1H), 6.88 (d, J = 8.9 Hz, 1H), 5.65 (s, 3H), 3.90 (d, J = 1.8 Hz, 3H), 3.57 (d, J = 1.9 Hz, 3H). |
| 52 | N-(3-(7-amino-1-methyl-2,4-dioxo-1,4-dihydropyrimido[4,5-d] pyrimidin-3 (2H) - yl)-2,4-difluorophenyl)-5-chloro-2-methylbenzenesulfonam ide | 1H NMR (400 MHz, CDCl3-d ) δ 8.95 (d, J = 1.8 Hz, 1H), 7.99 (d, J = 2.2 Hz, 1H), 7.68 (s, 1H), 7.59 (d, J = 6.0 Hz, 1H), 7.45 (dd, J = 8.3, 2.1 Hz, 1H), 7.28 (s, 1H), 7.05 (t, J = 9.1 Hz, 1H), 6.05 (s, 1H), 5.66 (s, 2H), 3.59 (s, 3H), 2.59 (s, 3H). |
| 53 | N-(3-(7-amino-1-methyl-2,4-dioxo-1,4-dihydropyrimido[4,5-d]pyrimidin-3(2H)-yl)-2,4-difluorophenyl)-1-methyl-2-oxoindoline-5-sulfonamide | 1H NMR (300 MHz, DMSO-d) δ 10.21 (s, 1H), 8.70 (s, 1H), 7.96 (d, J = 7.6 Hz, 2H), 7.68 (d, J = 8.4 Hz, 1H), 7.49 - 7.33 (m, 2H), 7.27 (d, J = 9.4 Hz, 1H), 3.63 (s, 2H), 3.41 (s, 3H), 3.14 (s, 3H). |
| 54 | N-(3-(7-amino-1-methyl-2,4-dioxo-1,4-dihydropyrimido[4,5-d]pyrimidin-3(2H)-yl)-2,4-difluorophenyl)-2,5-difluorobenzenesulfon amide | 1H NMR (400 MHz, CDCl3-d) δ 8.93 (d, J = 1.9 Hz, 1H), 7.68 (d, J = 7.2 Hz, 1H), 7.55 (s, 1H), 7.21 (t, J = 9.0 Hz, 1H), 7.11 - 7.02 (m, 1H), 5.69 (s, 2H), 3.59 (t, J = 1.7 Hz, 3H). |
| 55 | N-(3-(7-amino-1-methyl-2,4-dioxo-1,2-dihydropyrimido[4,5-d]pyrimidin-3(4H)-yl)-2,4-difluorophenyl)-2,4-dichlorobenzenesulfon amide | 1H NMR (400 MHz, CDCl3-d) δ 8.94 (s, 1H), 7.91 (d, J = 8.4 Hz, 1H), 7.65 (d, J = 6.9 Hz, 1H), 7.55 (d, J = 2.1 Hz, 1H), 7.41 - 7.34 (m, 1H), 7.04 (t, J = 8.6 Hz, 1H), 5.69 (s, 2H), 3.58 (d, J = 1.9 Hz, 3H). |
| 56 | N-(3-(7-amino-1-methyl-2,4-dioxo-1,4-dihydropyrimido[4,5-d] pyrimidin-3 (2H) - yl)-2,4-difluorophenyl)-1-methyl-1H-pyrazole-4-sulfonamide | 1H NMR (400 MHz, CDCl3-d) δ 8.90 (d, J = 1.7 Hz, 1H), 7.73 (d, J = 10.7 Hz, 2H), 7.46 (s, 1H), 7.11 (t, J = 9.0 Hz, 1H), 6.71 (s, 1H), 5.69 (s, 2H), 3.91 (s, 3H), 3.57 (s, 3H). |
| 57 | N-(3-(7-amino-1-methyl-2,4-dioxo-1,4-dihydropyrimido[4,5-d] pyrimidin-3 (2H) - yl)-2,4-difluorophenyl)-2,5-dichloropyridine-3-sulfonamide | 1H NMR (300 MHz, CDCl3-d) δ 8.92 (s, 1H), 8.51 (d, J = 2.5 Hz, 1H), 8.27 - 8.20 (m, 2H), 7.67 (td, J = 8.9, 5.4 Hz, 1H), 7.07 (td, J = 8.9, 2.0 Hz, 1H), 5.70 (s, 2H), 3.56 (s, 3H). |
| 58 | 5-chloro-N-(2,4-difluoro-3-(1-methyl-7-(methylamino)-2,4-dioxo-1,4-dihydropyrimido[4,5-d]pyrimidin-3(2H)-yl)phenyl)-2-methoxypyridine-3-sulfonamide | ¹H NMR (400 MHz, CDCl₃) δ 8.87 (s, 1H), 8.29 (s, 1H), 8.09 (s, 1H), 7.68 (d, J = 7.3 Hz, 2H), 7.58 (s, 1H), 7.04 (t, J = 9.1 Hz, 1H), 6.03 (s, 1H), 4.08 (s, 3H), 3.65 (s, 3H), 3.15 (s, 3H). |
| 59 | N-(3-(7-amino-1-cyclopropyl-4-oxo-1,4-dihydropyrimido[4,5-d]pyrimidin-3(2H)-yl)-2,4-difluorophenyl)-2,5-dichlorobenzenesulfon amide | 1H-NMR (300 MHz, CDCl3) δ 8.60 (s, 1H), 7.99 (t, J = 1.4 Hz, 1H), 7.49 (m, 3H), 6.97 (td, J = 9.2, 2.0 Hz, 1H), 5.59 (br, 2H), 4.84 (d, J = 9.6 Hz, 1H), 4.73 (d, J = 9.6 Hz, 1H), 2.56 (m, 1H), 0.93 (m, 2H), 0.73 (m, 2H). |
| 60 | N-(3-(7-amino-1-cyclopropyl-4-oxo-1,4-dihydropyrimido[4,5-d]pyrimidin-3(2H)-yl)-2,4-difluorophenyl)-5-chloro-2-methoxypyridine-3-sulfonamide | 1H-NMR (300 MHz, CDCl3) δ 8.63 (s, 1H), 8.28 (d, J = 2.6 Hz, 1H), 8.06 (d, J = 2.6 Hz, 1H), 7.54 (dt, J = 8.9, 4.4 Hz, 1H), 6.97 (td, J = 9.2, 2.0 Hz, 1H), 5.58 (br, 2H), 4.84 (d, J = 9.5 Hz, 1H), 4.72 (d, J = 9.6 Hz, 1H), 4.08 (s, 3H), 2.56 (m, 1H), 0.92 (m, 2H), 0.73 (m, 2H). |
| 61 | N-(3-(7-amino-1-cyclopropyl-4-oxo-1,4-dihydropyrimido[4,5-d]pyrimidin-3(2H)-yl)-2,4-difluorophenyl)naphth alene-1-sulfonamide | 1H-NMR (300 MHz, CDCl3) δ 8.64 (m, 1H), 8.58 (s, 1H), 8.23 (dd, J = 7.4, 1.2 Hz, 1H), 8.08 (d, J = 8.2 Hz, 1H), 7.93 (m, 1H), 7.60 (m, 2H), 7.51 (dd, J = 8.2, 7.4 Hz, 1H), 7.42 (td, J = 8.9, 5.4 Hz, 1H), 6.89 (td, J = 9.2, 2.0 Hz, 1H), 5.58 (br, 2H), 4.64 (d, J = 9.7 Hz, 1H), 4.46 (d, J = 9.4 Hz, 1H), 2.50 (m, 1H), 0.88 (m, 2H), 0.66 (m, 2H). |
| 62 | N-(3-(7-amino-1-cyclopropyl-4-oxo-1,4-dihydropyrimido[4,5-d]pyrimidin-3(2H)-yl)-2,4-difluorophenyl)-5-chloro-1,3-dimethyl-1H-pyrazole-4-sulfonamide | 1H-NMR (300 MHz, CDCl3) δ 8.63 (s, 3H), 7.55 (td, J = 8.9, 5.5 Hz, 1H), 7.39 (br, 1H), 6.98 (td, J = 9.2, 2.0 Hz, 1H), 5.42 (br, 2H), 4.86 (d, J = 9.1 Hz, 1H), 4.75 (d, J = 9.5 Hz, 1H), 3.79 (s, 3H), 2.56 (m, 1H), 2.30 (s, 3H), 0.93 (m, 2H), 0.76 (m, 2H). |
| 63 | N-(3-(7-amino-1-cyclopropyl-2,4-dioxo-1,4-dihydropyrimido[4,5-d]pyrimidin-3(2H)-yl)-2,4-difluorophenyl)-2,5-dichlorobenzenesulfon amide | 1H NMR (300 MHz, CDCl3) δ 8.92 (s, 1H), 8.02 (dd, J = 2.2, 0.7 Hz, 1H), 7.66 (td, J = 8.9, 5.4 Hz, 2H), 7.55 - 7.42 (m, 3H), 7.04 (td, J = 9.0, 2.0 Hz, 1H), 5.76 (s, 3H), 2.84 (dq, J = 7.3, 3.6 Hz, 1H), 1.31 - 1.20 (m, 2H), 0.96 - 0.88 (m, 2H). |
| 64 | N-(3-(7-amino-1-cyclopropyl-2,4-dioxo-1,4-dihydropyrimido[4,5-d]pyrimidin-3(2H)-yl)-2,4-difluorophenyl)naphth alene-1-sulfonamide | 1H NMR (300 MHz, CDCl3) δ 8.86 (s, 1H), 8.68 (d, J = 8.5 Hz, 1H), 8.19 (dd, J = 7.4, 1.2 Hz, 1H), 8.09 (d, J = 8.3 Hz, 1H), 7.95 (d, J = 8.0 Hz, 1H), 7.74 - 7.46 (m, 3H), 7.31 (s, 1H), 6.96 (td, J = 9.1, 2.1 Hz, 1H), 5.75 (s, 2H), 2.79 (dt, J = 7.1, 3.4 Hz, 1H), 1.30 - 1.13 (m, 2H), 0.95 - 0.77 (m, 2H). |
| 65 | N-(3-(7-amino-1-cyclopropyl-2,4-dioxo-1,4-dihydropyrimido[4,5-d]pyrimidin-3(2H)-yl)-2,4-difluorophenyl)-5-chloro-2-methoxypyridine-3-sulfonamide | 1H NMR (300 MHz, CDCl3) δ 8.90 (s, 1H), 8.26 (d, J = 2.6 Hz, 1H), 8.05 (d, J = 2.6 Hz, 1H), 7.75 - 7.48 (m, 2H), 7.01 (td, J = 9.0, 2.0 Hz, 1H), 5.73 (s, 2H), 4.04 (s, 3H), 2.82 (dt, J = 7.1, 3.4 Hz, 1H), 1.22 (dt, J = 6.8, 3.3 Hz, 2H), 0.94 - 0.84 (m, 2H). |
| 66 | N-(3-(7-amino-1-cyclopropyl-2,4-dioxo-1,4-dihydropyrimido[4,5-d]pyrimidin-3(2H)-yl)-2,4-difluorophenyl)-5-chloro-1,3-dimethyl-1H-pyrazole-4-sulfonamide | 1H NMR (400 MHz, CDCl3) δ 8.89 (s, 1H), 7.67 (d, J = 9.0 Hz, 1H), 7.04 (d, J = 9.0 Hz, 1H), 6.88 (s, 1H), 5.69 (s, 3H), 3.80 (s, 3H), 2.83 (d, J = 7.6 Hz, 1H), 2.34 (s, 3H), 1.22 (d, J = 6.7 Hz, 2H), 0.94 - 0.84 (m, 3H). |

The structures of the compounds of Examples 1 to 66 are as shown in Table 3 below.

**[Table 3]**

| Exa mpl e | Compound Structure | Exa mpl e | Compound Structure |
|---|---|---|---|
| 1 | 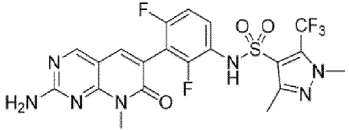 | 34 | 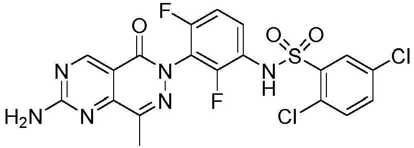 |
| 2 | 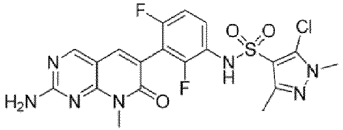 | 35 | 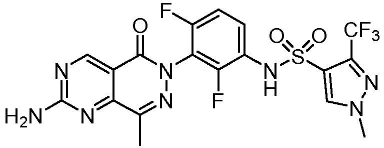 |
| 3 | 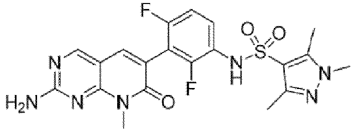 | 36 | 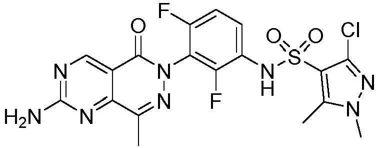 |
| 4 | 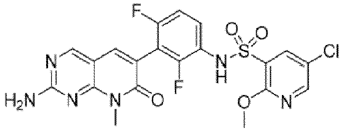 | 37 | 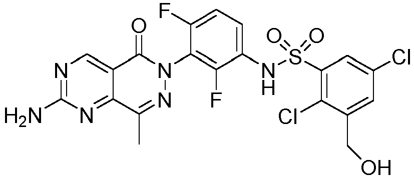 |
| 5 | 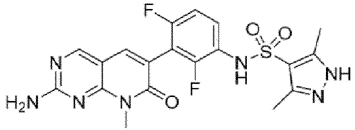 | 38 | 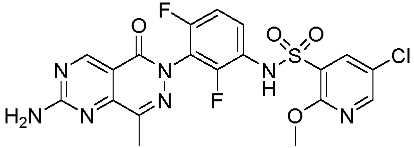 |
| 6 | 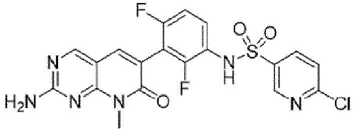 | 39 | 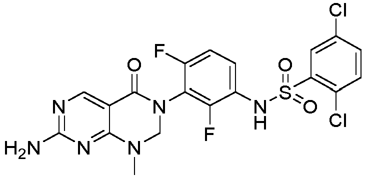 |
| 7 | 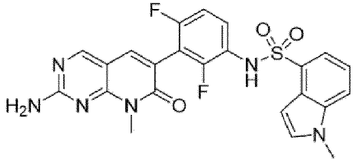 | 40 | 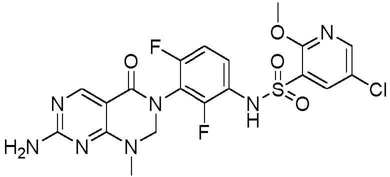 |
| 8 | 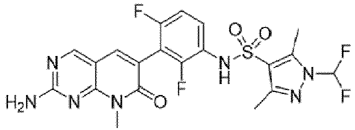 | 41 | 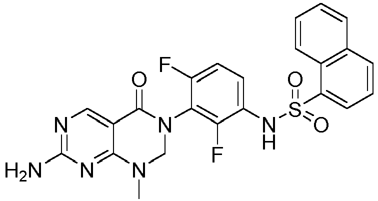 |
| 9 | 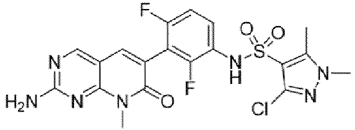 | 42 | 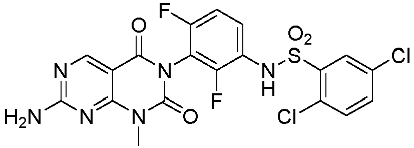 |
| 10 | 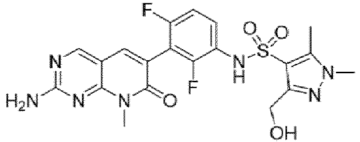 | 43 | 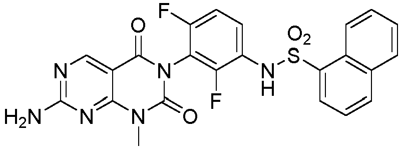 |
| 11 | 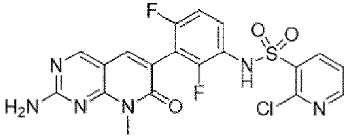 | 44 | 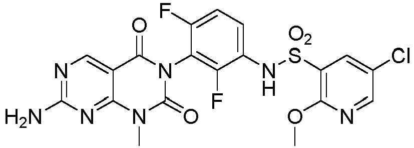 |
| 12 | 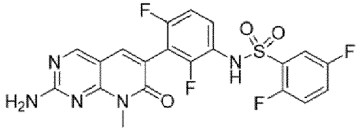 | 45 | 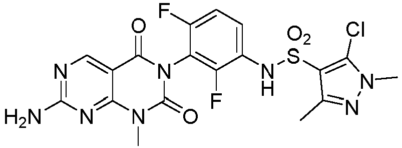 |
| 13 | 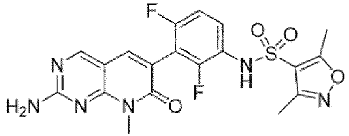 | 46 | 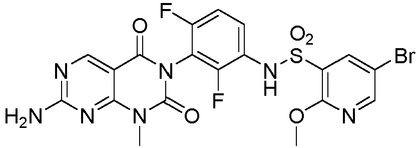 |
| 14 | 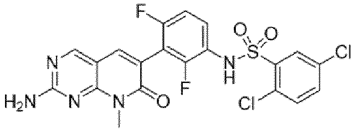 | 47 | 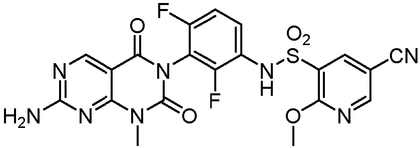 |
| 15 | 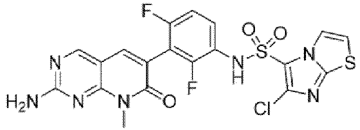 | 48 | 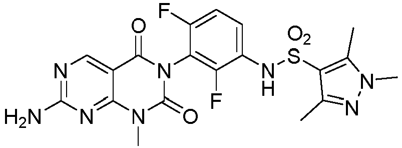 |
| 16 | 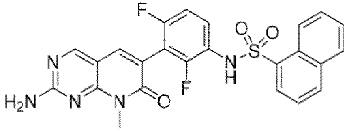 | 49 | 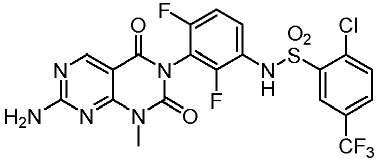 |
| 17 | 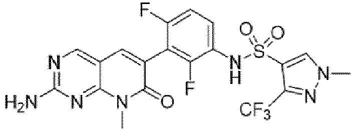 | 50 | 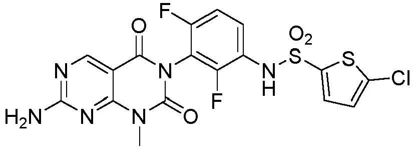 |
| 18 | 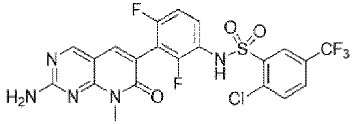 | 51 | 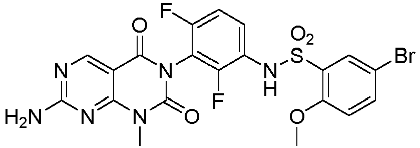 |
| 19 | 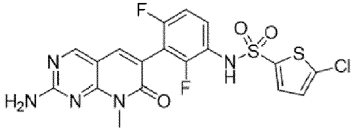 | 52 | 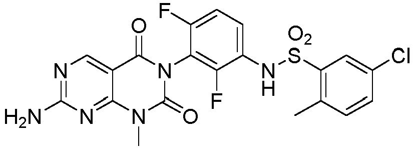 |
| 20 | 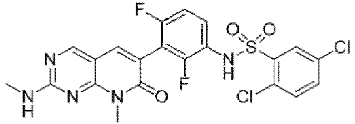 | 53 | 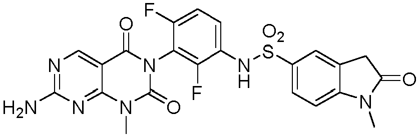 |
| 21 | 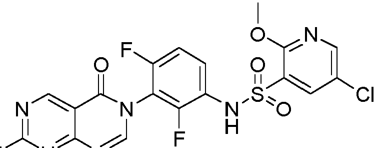 | 54 | 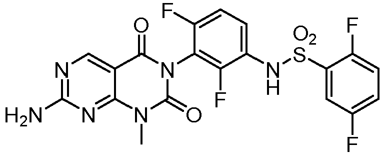 |
| 22 | 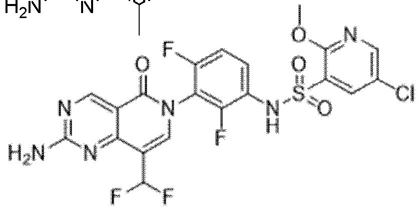 | 55 | 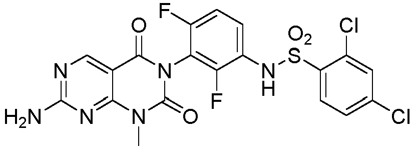 |
| 23 | 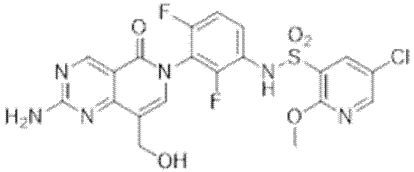 | 56 | 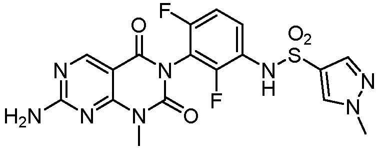 |
| 24 | 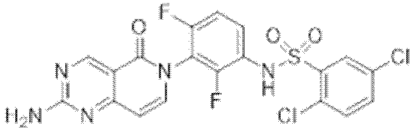 | 57 | 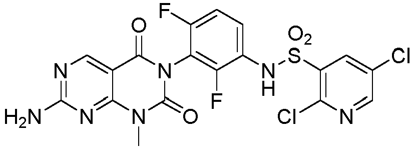 |
| 25 | 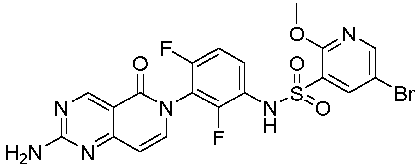 | 58 | 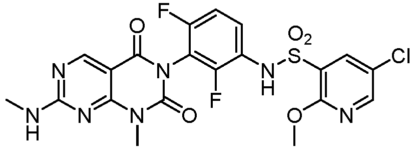 |
| 26 | 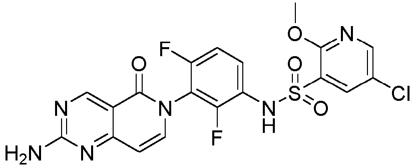 | 59 | 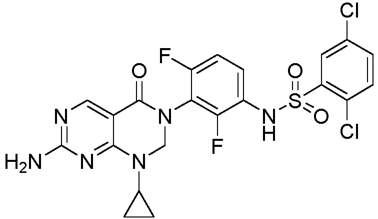 |
| 27 | 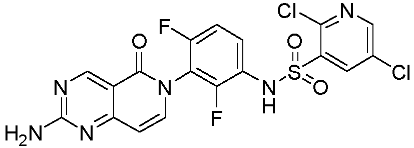 | 60 | 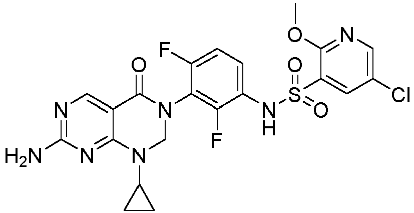 |
| 28 | 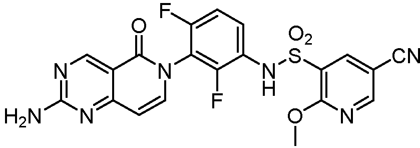 | 61 | 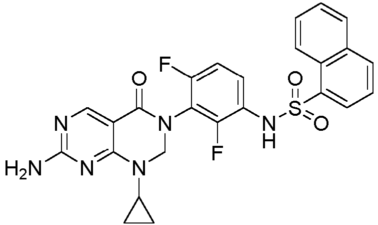 |
| 29 | 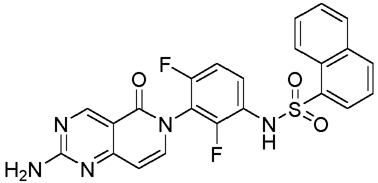 | 62 | 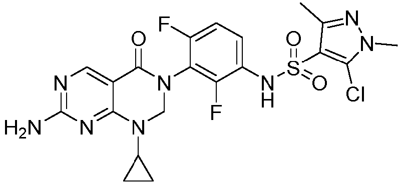 |
| 30 | 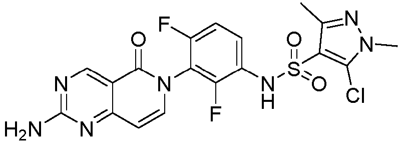 | 63 | 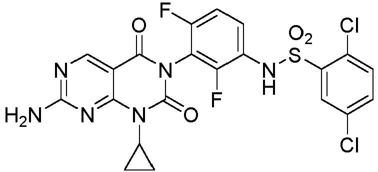 |
| 31 | 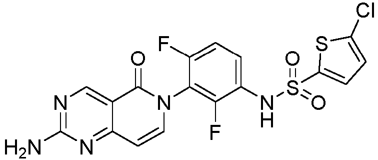 | 64 | 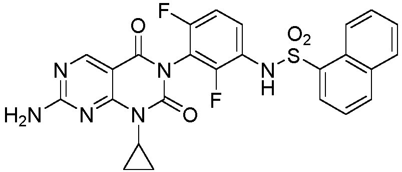 |
| 32 | 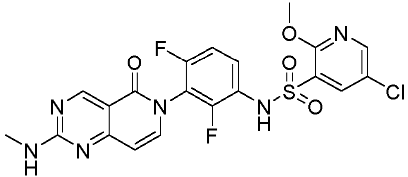 | 65 | 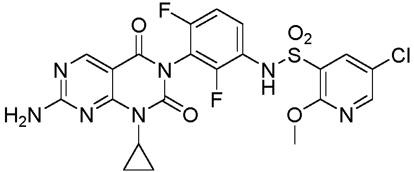 |
| 33 | 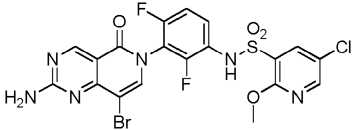 | 66 | 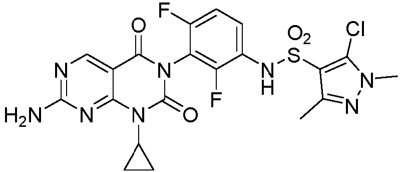 |

### <Experimental Example 1> Evaluation of PKR-like endoplasmic reticulum kinase (PERK) inhibitory capacity

A recombinant human PERK protein was mixed with the compound in a reaction solution (8 mM MOPS pH 7.0, 0.2 mM EDTA, 300 uM RSRSRSRSRSRSRSR, 10 mM Magnesium acetate, 15 uM [gamma-33P-ATP]) and reacted at room temperature for 40 minutes. Then, the reaction was terminated by adding phosphoric acid (0.5%) to the reaction mixture, and 10 ul of the reaction solution was dropped onto a P30 filter. The filter was washed four times in a 0.425% phosphoric acid solution for 4 minutes, then washed once more in methanol, dried, and the PERK activity was measured by scintillation counting. The activity value obtained in the group that did not contain the compound was set as 100% of control to calculate the degree of PERK inhibition by the compound. The compound was diluted by 1/3 from the highest concentration of 1 uM and treated with a total of 9 concentrations to evaluate the degree of inhibition. The concentration that resulted in 50% inhibition was defined as the IC₅₀ value and is summarized in Table 3.

### <Experimental Example 2> Evaluation of general control nonderepressible 2 (GCN2) inhibitory capacity

A recombinant human GCN2 protein was mixed with the compound in a reaction solution (20 mM Tris/HCl pH8.5, 0.2 mM EDTA, 0.1% Tween, 100 mM NaCl, 300 uM RSRSRSRSRSRSRSR, 10 mM Magnesium acetate, 70 uM [gamma-33P-ATP]) and reacted at room temperature for 40 minutes. Then, the reaction was terminated by adding phosphoric acid (0.5%) to the reaction mixture, and 10 ul of the reaction solution was dropped onto a P30 filter. The filter was washed four times in a 0.425% phosphoric acid solution for 4 minutes, then washed once more in methanol, dried, and the GCN2 activity was measured by scintillation counting. The activity value obtained in the group that did not contain the compound was set as 100% of control to calculate the degree of GCN2 inhibition by the compound. The compound was diluted by 1/3 from the highest concentration of 1 uM and treated with a total of 9 concentrations to evaluate the degree of inhibition. The concentration that resulted in 50% inhibition was defined as the IC₅₀ value and is summarized in Table 3.

### <Experimental Example 3> Evaluation of eIF2-alpha kinase heme-regulated inhibitor (HRI) inhibitory capacity

A recombinant human HRI protein was mixed with the compound in a reaction solution (50 mM Tris/HCl pH 7.5, 0.04 mM EDTA, 300 uM RSRSRSRSRSRSRSR, 10 mM Magnesium acetate, 45 uM [gamma-33P-ATP]) and reacted at room temperature for 40 minutes. Then, the reaction was terminated by adding phosphoric acid (0.5%) to the reaction mixture, and 10 ul of the reaction solution was dropped onto a P30 filter. The filter was washed four times in a 0.425% phosphoric acid solution for 4 minutes, then washed once more in methanol, dried, and the HRI activity was measured by scintillation counting. The activity value obtained in the group that did not contain the compound was set as 100% of control to calculate the degree of HRI inhibition by the compound. The compound was diluted by 1/3 from the highest concentration of 1 uM and treated with a total of 9 concentrations to evaluate the degree of inhibition. The concentration that resulted in 50% inhibition was defined as the IC₅₀ value and is summarized in Table 3.

### <Experimental Example 4> Evaluation of protein kinase R (PKR) inhibitory capacity

A recombinant human PKR protein was mixed with the compound in a reaction solution (8 mM MOPS pH 7.0, 0.2 mM EDTA, 20 uM RSRSRSRSRSRSRSR, 10 mM Magnesium acetate, 15 uM [gamma-33P-ATP]) and reacted at room temperature for 40 minutes. Then, the reaction was terminated by adding phosphoric acid (0.5%) to the reaction mixture, and 10 ul of the reaction solution was dropped onto a P30 filter. The filter was washed four times in a 0.425% phosphoric acid solution for 4 minutes, then washed once more in methanol, dried, and the PKR activity was measured by scintillation counting. The activity value obtained in the group that did not contain the compound was set as 100% of control to calculate the degree of PKR inhibition by the compound. The compound was diluted by 1/3 from the highest concentration of 1 uM and treated with a total of 9 concentrations to evaluate the degree of inhibition. The concentration that resulted in 50% inhibition was defined as the IC₅₀ value and is summarized in Table 3.

The measured results of Experiment Examples 1 to 4 are summarized in Table 3 below (IC₅₀ value: +++ <100 nM; ++ 100-300 nM; + 300-1,000 nM; - >1,000 nM).

**[Table 3]**

| Example | PERK(nM) | GCN2(nM) | HRI(nM) | PKR(nM) |
|---|---|---|---|---|
| 1 | + | ++ | | |
| 2 | + | | | |
| 3 | | + | | |
| 4 | +++ | | | |
| 5 | | | | |
| 6 | | | | |
| 7 | +++ | ++ | | |
| 8 | | | | |
| 9 | | + | | |
| 10 | | | | |
| 11 | ++ | ++ | | |
| 12 | +++ | ++ | | |
| 13 | + | ++ | | |
| 14 | +++ | +++ | | |
| 15 | ++ | + | | |
| 16 | +++ | ++ | | |
| 17 | ++ | + | | |
| 18 | +++ | ++ | | |
| 19 | +++ | +++ | | |
| 20 | | ++ | | |
| 21 | | +++ | | |
| 22 | +++ | +++ | +++ | + |
| 23 | | ++ | | |
| 24 | | +++ | | |
| 25 | +++ | +++ | +++ | |
| 26 | | +++ | | |
| 27 | +++ | +++ | +++ | |
| 28 | +++ | +++ | ++ | |
| 29 | +++ | +++ | +++ | ++ |
| 30 | | ++ | | |
| 31 | | ++ | | |
| 32 | +++ | +++ | +++ | |
| 33 | | | | |
| 34 | | +++ | | |
| 35 | | | | |
| 36 | | ++ | | |
| 37 | | | | |
| 38 | | ++ | | |
| 39 | | ++ | | |
| 40 | | ++ | | |
| 41 | | ++ | | |
| 42 | | ++ | | |
| 43 | | ++ | | |
| 44 | +++ | ++ | +++ | + |
| 45 | | + | | |
| 46 | +++ | +++ | ++ | + |
| 47 | | +++ | | |
| 48 | | ++ | | |
| 49 | | ++ | | |
| 50 | | +++ | | |
| 51 | | ++ | | |
| 52 | | +++ | | |
| 53 | | | | |
| 54 | | ++ | | |
| 55 | | ++ | | |
| 56 | | | | |
| 57 | +++ | +++ | ++ | + |
| 58 | | ++ | | |
| 59 | | ++ | | |
| 60 | | ++ | | |
| 61 | | ++ | | |
| 62 | | | | |
| 63 | | + | | |
| 64 | | + | | |
| 65 | | + | | |
| 66 | | + | | |

### <Experimental Example 5> Evaluation of inhibitory efficacy of ISR-related kinase GCN2 under amino acid deficiency condition using Western blot

HT-1080 cells, a human fibrosarcoma cell line, were cultured in RPMI-1640 medium supplemented with 10% FBS, penicillin (penicillin; 100 IU/mℓ), and streptomycin (streptomycin; 100 *µ*g/mℓ). Cells (1×10⁶ cells/ml) were seeded onto 100 mm plates (polypropylene tissue culture plate) and incubated for 24 hours in a 37°C, 5% CO₂ incubator before being used for experiments.

The following method was used to achieve amino acid deficiency conditions in cells. After 24 hours of cell culture, the cells were cultured in amino acids and L-glutamine-free RPMI-1640 medium supplemented with 10% FBS, penicillin (100 IU/mℓ), and streptomycin (100 *µ*g/mℓ). After 24 hours of cell culture in RPMI-1640 without amino acids and L-glutamine, the compound prepared at a concentration of 50 nM was dispensed onto each plate and cultured for 24 hours in a 37°C, 5% CO₂ incubator.

In order to measure the signal transduction inhibitory efficacy of GCN among the ISR-related kinases according to the present invention, the following experiment was performed using SDS-PAGE. The fibrosarcoma cell line HT-1080 cells cultured on plates as described above were washed with PBS after removing the culture medium. After removing PBS, the cells were lysed by adding a radio-immunoprecipitation assay buffer (RIPA buffer) containing 50 mM Tris (pH 7.4), 150 mM NaCl, 1% NP-40, 0.5% sodium deoxycholate, 2 mM sodium fluoride, 2 mM EDTA, 0.1% SDS, and a protease/phosphatase inhibitor cocktail to the plate. After centrifugation at 4°C, 13,000×g for 15 minutes, the protein in the upper layer was collected, and the protein concentration was measured at 562 nm using BCA (bicinchoninc acid) reagent, and the amount of protein in all samples was prepared at 20-50 µg.

After making the total volume the same using the sample buffer and RIPA buffer, it was heated at 97°C for 5 minutes. Then, electrophoresis was performed on 8% or 12% SDS-PAGE at 80 V for 30 minutes and at 160 V for 1 hour and 30 minutes. After electrophoresis, the SDS-PAGE gel was transferred to an NC membrane (nitrocellulose membrane) at 90 V for 2 hours. The NC membrane was placed in TBST (tris buffer saline tween-20; 20 mM tris, 137 mM sodium chloride, 0.1% tween-20, pH 8.0) containing 5% non-fat skim milk, placed on a shaker, and reacted at room temperature for 1 hour.

Each primary antibody was diluted in TBST containing 5% non-fat skim milk, poured onto the NC membrane, and reacted at 4°C for 24 hours. The membrane was washed three times for 10 minutes each with TBST. The secondary antibodies (anti-rabbit IgG and anti-mouse IgG labeled with horseradish peroxidase (HRP)) were diluted in TBST containing 5% non-fat skim milk and reacted with the membrane at room temperature for 1 hour. Upon completion of the reaction, the membrane was washed three times with TBST for 10 minutes each. Protein expression bands were detected by a chemiluminescence image analyzer (Image Quant LAS 500) after the ECL solution was reacted with the NC membrane.

GCN2, a key kinase of the integrated stress response (ISR), is phosphorylated in response to amino acid deficiency. Therefore, when the inhibitory effect on the phosphorylated GCN2 was confirmed during the treatment of the compounds, the compounds (Examples 25, 26, 27, 29, 32, 36, 43, 44, 46, 51, 63, 64, and 65) showed an excellent inhibitory effect on the phosphorylated GCN2 (Table 4).

Figure 1 is a set of photographs confirming the mechanism proteins that react upon ISR activation, wherein the numbers correspond to example numbers of the present invention. The four major specialized kinases of ISR (PERK, GCN2, PKR, and HRI) commonly phosphorylate serine 52 of eIF2α in response to stress, leading to an increase in certain transcription factors (notably ATF4). They are also known to have an effect on increasing the expression of CHOP (GADD153), the most downstream factor of the mechanism. The compounds synthesized in the above examples (Examples 22, 25, 29, 43, 44, 46, and 51) exhibited excellent inhibitory effects not only on GCN2 but also on ATF4, a downstream factor of the major kinase of the integrated stress response (Figure 1). Thus, in addition to the activation of ISR by amino acid deficiency, a significant effect can be expected on the mechanism of inhibition of the activity of PERK, an ISR-related kinase, due to endoplasmic reticulum stress response.

**[Table 4]**

| Example | Phospho-GCN2 inhibition | Example | Phospho-GCN2 inhibition |
|---|---|---|---|
| 21 | X | 43 | O |
| 22 | O | 44 | O |
| 25 | O | 45 | O |
| 26 | O | 46 | O |
| 27 | O | 47 | X |
| 29 | O | 48 | O |
| 30 | X | 51 | O |
| 31 | X | 57 | X |
| 32 | O | 63 | O |
| 35 | X | 64 | O |
| 36 | X | 65 | O |
| 38 | O | 66 | X |

## Claims

1. A compound represented by Formula 1 below, a stereoisomer thereof, a solvate thereof, a hydrate thereof, or a pharmaceutically acceptable salt thereof: 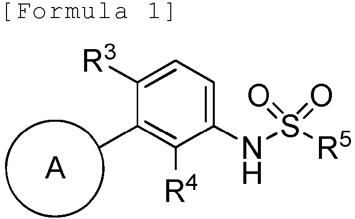 in Formula 1,
A is 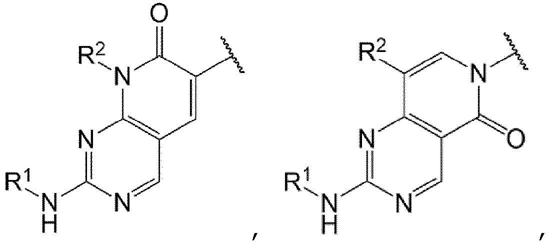 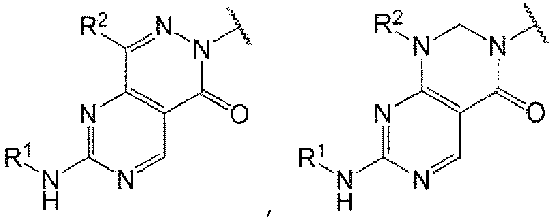
or 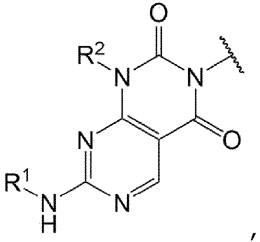
R¹ is hydrogen or C1-C10 alkyl;
R² is hydrogen, halogen, C1-C10 alkyl unsubstituted or substituted with halogen or hydroxyl, or C3-C10 cycloalkyl;
R³ and R⁴ are independently halogen;
R⁵ is unsubstituted or substituted C6-C10 aryl, or unsubstituted or substituted 5-10 membered heteroaryl,
wherein, the substituted C6-C10 aryl and the substituted 5-10 membered heteroaryl are independently substituted with at least one selected from the group consisting of C1-C10 alkyl unsubstituted or substituted with halogen or hydroxyl, C1-C10 alkoxy, halogen, and cyano, or substituted for two adjacent atoms to form 5-6 membered heterocycle. At this time, the 5-6 membered heterocycle contains at least one N, and is unsubstituted or substituted with at least one selected from C1-C10 alkyl and oxo.

2. The compound, the stereoisomer thereof, the solvate thereof, the hydrate thereof, or the pharmaceutically acceptable salt thereof according to claim 1, wherein:
R¹ is hydrogen or C1-C6 alkyl;
R² is hydrogen, halogen, C1-C6 alkyl unsubstituted or substituted with 1 to 3 halogens or hydroxyls, or C3-C8 cycloalkyl;
R³ and R⁴ are independently halogen;
R⁵ is unsubstituted or substituted C6-C10 aryl, or unsubstituted or substituted 5-9 membered heteroaryl,
wherein, the substituted C6-C10 aryl and the substituted 5-9 membered heteroaryl are independently substituted with 1 to 3 substituents selected from the group consisting of C1-C6 alkyl unsubstituted or substituted with 1 to 3 halogens or hydroxyls, C1-C6 alkoxy, halogen, and cyano, or substituted for two adjacent atoms to form 5-6 membered heterocycle. At this time, the 5-6 membered heterocycle contains at least one N, and is unsubstituted or substituted with 1 to 3 substituents selected from C1-C6 alkyl and oxo.

3. The compound, the stereoisomer thereof, the solvate thereof, the hydrate thereof, or the pharmaceutically acceptable salt thereof according to claim 1, wherein:
R¹ is hydrogen or C1-C4 alkyl;
R² is hydrogen, halogen, C1-C4 alkyl unsubstituted or substituted with 1 to 3 halogens or hydroxyls, or C3-C6 cycloalkyl;
R³ and R⁴ are independently halogen;
R⁵ is unsubstituted or substituted C6-C10 aryl, or unsubstituted or substituted 5-9 membered heteroaryl,
wherein, the substituted C6-C10 aryl and the substituted 5-9 membered heteroaryl are independently substituted with 1 to 3 substituents selected from the group consisting of C1-C4 alkyl unsubstituted or substituted with 1 to 3 Fs or hydroxyls, C1-C4 alkoxy, F, Cl, Br, and cyano, or substituted for two adjacent atoms to form 5 membered non-aromatic heterocycle. At this time, the 5 membered heterocycle contains at least one N, and is unsubstituted or substituted with 1 to 2 substituents selected from C1-C4 alkyl and oxo.

4. The compound, the stereoisomer thereof, the solvate thereof, the hydrate thereof, or the pharmaceutically acceptable salt thereof according to claim 1, wherein:
R¹ is hydrogen or methyl;
R² is hydrogen, halogen, methyl unsubstituted or substituted with 1 to 3 Fs or hydroxyls, or cyclopropyl;
R³ and R⁴ are independently F;
R⁵ is substituted phenyl, naphthalenyl, substituted pyridinyl, substituted pyrazolyl, substituted isoxazolyl, substituted thiophenyl, substituted indolyl, or substituted imidazothiazolyl,
wherein, the substituted phenyl, substituted pyridinyl, substituted pyrazolyl, substituted isoxazolyl, substituted thiophenyl, substituted indolyl, and substituted imidazothiazolyl are independently substituted with 1 to 3 substituents selected from the group consisting of methyl unsubstituted or substituted with 1 to 3 Fs or hydroxyls, ethyl, methoxy, F, Cl, Br, oxo, and cyano, or substituted for two adjacent atoms to form 5 membered non-aromatic heterocycle. At this time, the 5 membered heterocycle contains at least one N, and is substituted with 1 to 2 substituents selected from methyl and oxo.

5. The compound, the stereoisomer thereof, the solvate thereof, the hydrate thereof, or the pharmaceutically acceptable salt thereof according to claim 1, wherein:
R¹ is hydrogen or methyl;
R² is hydrogen, 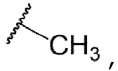 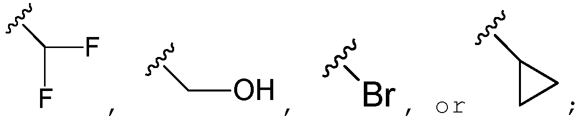
R⁵ is 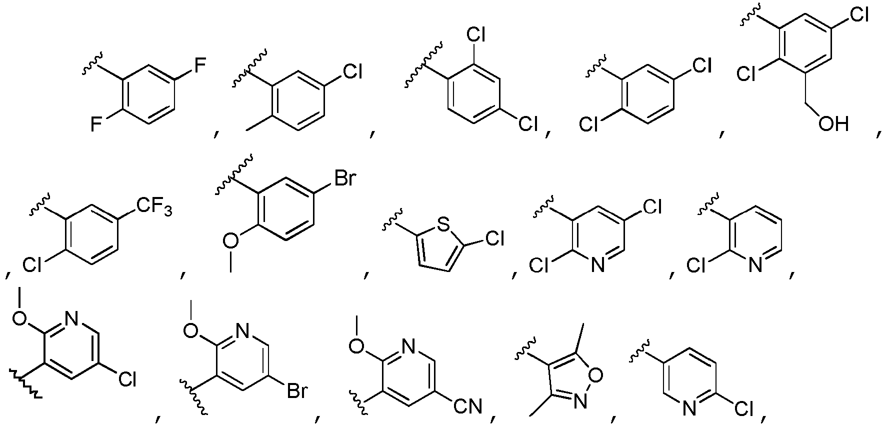 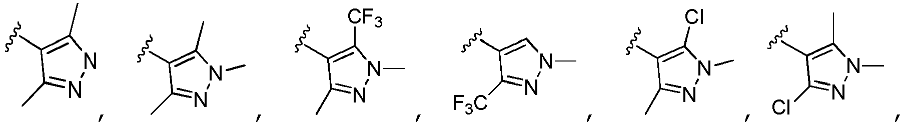 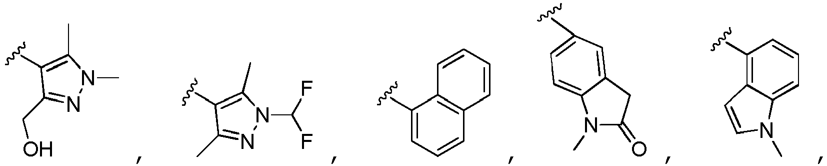 or

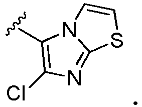

6. The compound, the stereoisomer thereof, the solvate thereof, the hydrate thereof, or the pharmaceutically acceptable salt thereof according to claim 1, wherein the compound is selected from the group consisting of the following compounds:
<1> N-(3-(2-amino-8-methyl-7-oxo-7,8-dihydropyrido[2,3-d]pyrimidin-6-yl)-2,4-difluorophenyl)-1,3-dimethyl-5-(trifluoromethyl)-1H-pyrazole-4-sulfonamide;
<2> N-(3-(2-amino-8-methyl-7-oxo-7,8-dihydropyrido[2,3-d]pyrimidin-6-yl)-2,4-difluorophenyl)-5-chloro-1,3-dimethyl-1H-pyrazole-4-sulfonamide;
<3> N-(3-(2-amino-8-methyl-7-oxo-7,8-dihydropyrido[2,3-d]pyrimidin-6-yl)-2,4-difluorophenyl)-1,3,5-trimethyl-1H-pyrazole-4-sulfonamide;
<4> N-(3-(2-amino-8-methyl-7-oxo-7,8-dihydropyrido[2,3-d]pyrimidin-6-yl)-2,4-difluorophenyl)-5-chloro-2-methoxypyridine-3-sulfonamide;
<5> N-(3-(2-amino-8-methyl-7-oxo-7,8-dihydropyrido[2,3-d]pyrimidin-6-yl)-2,4-difluorophenyl)-3,5-dimethyl-1H-pyrazole-4-sulfonamide;
<6> N-(3-(2-amino-8-methyl-7-oxo-7,8-dihydropyrido[2,3-d]pyrimidin-6-yl)-2,4-difluorophenyl)-6-chloropyridine-3-sulfonamide;
<7> N-(3-(2-amino-8-methyl-7-oxo-7,8-dihydropyrido[2,3-d]pyrimidin-6-yl)-2,4-difluorophenyl)-1-methyl-1H-indole-4-sulfonamide;
<8> N-(3-(2-amino-8-methyl-7-oxo-7,8-dihydropyrido[2,3-d]pyrimidin-6-yl)-2,4-difluorophenyl)-1-(difluoromethyl)-3,5-dimethyl-1H-pyrazole-4-sulfonamide;
<9> N-(3-(2-amino-8-methyl-7-oxo-7,8-dihydropyrido[2,3-d]pyrimidin-6-yl)-2,4-difluorophenyl)-3-chloro-1,5-dimethyl-1H-pyrazole-4-sulfonamide;
<10> N-(3-(2-amino-8-methyl-7-oxo-7,8-dihydropyrido[2,3-d]pyrimidin-6-yl)-2,4-difluorophenyl)-3-(hydroxymethyl)-1,5-dimethyl-1H-pyrazole-4-sulfonamide;
<11> N-(3-(2-amino-8-methyl-7-oxo-7,8-dihydropyrido[2,3-d]pyrimidin-6-yl)-2,4-difluorophenyl)-2-chloropyridine-3-sulfonamide;
<12> N-(3-(2-amino-8-methyl-7-oxo-7,8-dihydropyrido[2,3-d]pyrimidin-6-yl)-2,4-difluorophenyl)-2,5-difluorobenzene sulfonamide;
<13> N-(3-(2-amino-8-methyl-7-oxo-7,8-dihydropyrido[2,3-d]pyrimidin-6-yl)-2,4-difluorophenyl)-3,5-dimethylisoxazole-4-sulfonamide;
<14> N-(3-(2-amino-8-methyl-7-oxo-7,8-dihydropyrido[2,3-d]pyrimidin-6-yl)-2,4-difluorophenyl)-2,5-dichlorobenzenesulfonamide;
<15> N-(3-(2-amino-8-methyl-7-oxo-7,8-dihydropyrido[2,3-d]pyrimidin-6-yl)-2,4-difluorophenyl)-6-chloroimidazo[2,1-b]thiazole-5-sulfonamide;
<16> N-(3-(2-amino-8-methyl-7-oxo-7,8-dihydropyrido[2,3-d]pyrimidin-6-yl)-2,4-difluorophenyl)naphthalene-1-sulfonamide;
<17> N-(3-(2-amino-8-methyl-7-oxo-7,8-dihydropyrido[2,3-d]pyrimidin-6-yl)-2,4-difluorophenyl)-1-methyl-3-(trifluoromethyl)-1H-pyrazole-4-sulfonamide;
<18> N-(3-(2-amino-8-methyl-7-oxo-7,8-dihydropyrido[2,3-d]pyrimidin-6-yl)-2,4-difluorophenyl)-2-chloro-5-(trifluoromethyl)benzenesulfonamide;
<19> N-(3-(2-amino-8-methyl-7-oxo-7,8-dihydropyrido[2,3-d]pyrimidin-6-yl)-2,4-difluorophenyl)-5-chlorothiophene-2-sulfonamide;
<20> 2,5-dichloro-N-(2,4-difluoro-3-(8-methyl-2-(methylamino)-7-oxo-7,8-dihydropyrido[2,3-d]pyrimidin-6-yl)phenyl)benzenesulfonamide;
<21> N-(3-(2-amino-8-methyl-5-oxopirido[4,3-d]pyrimidin-6(5H)-yl)-2,4-difluorophenyl)-5-chloro-2-methoxypyridine-3-sulfonamide;
<22> N-(3-(2-amino-8-(difluoromethyl)-5-oxopirido[4,3-d]pyrimidin-6(5H)-yl)-2,4-difluorophenyl)-5-chloro-2-methoxypyridine-3-sulfonamide;
<23> N-(3-(2-amino-8-hydroxymethyl)-5-oxopirido[4,3-d]pyrimidin-6(5H)-2,4-difluorophenyl)-5-chloro-2-methoxypyridine-3-sulfonamide;
<24> N-(3-(2-amino-5-oxopirido[4,3-d]pyrimidin-6(5H)-yl)-2,4-difluorophenyl)-2,5-dichlorobenzenesulfonamide;
<25> N-(3-(2-amino-5-oxopirido[4,3-d]pyrimidin-6(5H)-yl)-2,4-difluorophenyl)-5-bromo-2-methoxypyridine-3-sulfonamide;
<2 6> N- (3- (2-amino-5-oxopirido[4,3-d]pyrimidin-6(5H)-yl)-2,4-difluorophenyl)-5-chloro-2-methoxypyridine-3-sulfonamide;
<27>N-(3-(2-amino-5-oxopirido[4,3-d]pyrimidin-6(5H)-yl)-2,4-difluorophenyl)-2,5-dichloropyridine-3-sulfonamide;
<28>N-(3-(2-amino-5-oxopirido[4,3-d]pyrimidin-6(5H)-yl)-2,4-difluorophenyl)-5-cyano-2-methoxypyridine-3-sulfonamide;
<29> N-(3-(2-amino-5-oxopirido[4,3-d]pyrimidin-6(5H)-yl)-2,4-difluorophenyl)naphthalene-1-sulfonamide;
<30> N-(3-(2-amino-5-oxopirido[4,3-d]pyrimidin-6(5H)-yl)-2,4-difluorophenyl)-5-chloro-1,3-dimethyl-1H-pyrazole-4-sulfonamide;
<31> N- (3- (2-amino-5-oxopirido[4,3-d]pyrimidin-6(5H)-yl)-2,4-difluorophenyl)-5-chlorothiophene-2-sulfonamide;
<32> 5-chloro-N-(2,4-difluoro-3-(2-(methylamino)-5-oxopirido[4,3-d]pyrimidin-6(5H)-yl)phenyl)-2-methoxypyridine-3-sulfonamide;
<33> N- (3- (2-amino-8-bromo-5-oxopirido[4,3-d]pyrimidin-6(5H)-yl)-2,4-difluorophenyl)-5-chloro-2-methoxypyridine-3-sulfonamide;
<34> N- (3- (2-amino-8-methyl-5-oxopyrimido[4,5-d]pyridazine-6(5H)-yl)-2,4-difluorophenyl)-2,5-dichlorobenzenesulfonamide;
<35> N-(3-(2-amino-8-methyl-5-oxopyrimido[4,5-d]pyridazine-6(5H)-yl)-2,4-difluorophenyl)-1-methyl-3-(trifluoromethyl)-1H-pyrazole-4-sulfonamide;
<36> N- (3- (2-amino-8-methyl-5-oxopyrimido[4,5-d]pyridazine-6(5H)-yl)-2,4-difluorophenyl)-3-chloro-1,5-dimethyl- 1H-pyrazole-4-sulfonamide;
<37> N- (3- (2-amino-8-methyl-5-oxopyrimido[4,5-d]pyridazine-6(5H)-yl)-2,4-difluorophenyl)-2,5-dichloro-3-(hydroxymethyl)benzene sulfonamide;
<38> N-(3-(2-amino-8-methyl-5-oxopyrimido[4,5-d]pyridazine-6(5H)-yl)-2,4-difluorophenyl)-5-chloro-2-methoxypyridine-3-sulfonamide;
<39> N-(3-(7-amino-1-methyl-4-oxo-1,4-dihydropyrimido[4,5-d]pyrimidin-3(2H)-yl)-2,4-difluorophenyl)-2,5-dichlorobenzenesulfonamide;
<40> N-(3-(7-amino-1-methyl-4-oxo-1,4-dihydropyrimido[4,5-d]pyrimidin-3(2H)-yl)-2,4-difluorophenyl)-5-chloro-2-methoxypyridine-3-sulfonamide;
<41> N-(3-(7-amino-1-methyl-4-oxo-1,4-dihydropyrimido[4,5-d]pyrimidin-3(2H)-yl)-2,4-difluorophenyl)naphthalene-1-sulfonamide;
<42> N-(3-(7-amino-1-methyl-2,4-dioxo-1,4-dihydropyrimido[4,5-d]pyrimidin-3(2H)-yl)-2,4-difluorophenyl)-2,5-dichlorobenzenesulfonamide;
<43> N-(3-(7-amino-1-methyl-2,4-dioxo-1,4-dihydropyrimido[4,5-d]pyrimidin-3(2H)-yl)-2,4-difluorophenyl)naphthalene-1-sulfonamide;
<44> N-(3-(7-amino-1-methyl-2,4-dioxo-1,4-dihydropyrimido[4,5-d]pyrimidin-3(2H)-yl)-2,4-difluorophenyl)-5-chloro-2-methoxypyridine-3-sulfonamide;
<45> N-(3-(7-amino-1-methyl-2,4-dioxo-1,4-dihydropyrimido[4,5-d]pyrimidin-3(2H)-yl)-2,4-difluorophenyl)-5-chloro-2-methoxypyridine-3-sulfonamide;
<46> N-(3-(7-amino-1-methyl-2,4-dioxo-1,4-dihydropyrimido[4,5-d]pyrimidin-3(2H)-yl)-2,4-difluorophenyl)-5-bromo-2-methoxypyridine-3-sulfonamide;
<47> N-(3-(7-amino-1-methyl-2,4-dioxo-1,4-dihydropyrimido[4,5-d]pyrimidin-3(2H)-yl)-2,4-difluorophenyl)-5-cyano-2-methoxypyridine-3-sulfonamide;
<48> N-(3-(7-amino-1-methyl-2,4-dioxo-1,4-dihydropyrimido[4,5-d]pyrimidin-3(2H)-yl)-2,4-difluorophenyl)-1,3,5-trimethyl-1H-pyrazole-4-sulfonamide;
<49> N-(3-(7-amino-1-methyl-2,4-dioxo-1,2-dihydropyrimido[4,5-d]pyrimidin-3(4H)-yl)-2,4-difluorophenyl)-2-chloro-5-(trifluoromethyl)benzenesulfonamide;
<50> N-(3-(7-amino-1-methyl-2,4-dioxo-1,4-dihydropyrimido[4,5-d]pyrimidin-3(2H)-yl)-2,4-difluorophenyl)-5-chlorothiophene-2-sulfonamide;
<51> N-(3-(7-amino-1-methyl-2,4-dioxo-1,2-dihydropyrimido[4,5-d]pyrimidin-3(4H)-yl)-2,4-difluorophenyl)-5-bromo-2-methoxybenzenesulfonamide;
<52> N-(3-(7-amino-1-methyl-2,4-dioxo-1,4-dihydropyrimido[4,5-d]pyrimidin-3(2H)-yl)-2,4-difluorophenyl)-5-chloro-2-methylbenzenesulfonamide;
<53> N-(3-(7-amino-1-methyl-2,4-dioxo-1,4-dihydropyrimido[4,5-d]pyrimidin-3(2H)-yl)-2,4-difluorophenyl)-1-methyl-2-oxoindoline-5-sulfonamide;
<54> N-(3-(7-amino-1-methyl-2,4-dioxo-1,4-dihydropyrimido[4,5-d]pyrimidin-3(2H)-yl)-2,4-difluorophenyl)-2,5-difluorobenzenesulfonamide;
<55> N-(3-(7-amino-1-methyl-2,4-dioxo-1,2-dihydropyrimido[4,5-d]pyrimidin-3(4H)-yl)-2,4-difluorophenyl)-2,4-dichlorobenzenesulfonamide;
<56> N-(3-(7-amino-1-methyl-2,4-dioxo-1,4-dihydropyrimido[4,5-d]pyrimidin-3(2H)-yl)-2,4-difluorophenyl)-1-methyl-1H-pyrazole-4-sulfonamide;
<57> N-(3-(7-amino-1-methyl-2,4-dioxo-1,4-dihydropyrimido[4,5-d]pyrimidin-3(2H)-yl)-2,4-difluorophenyl)-2,5-dichloropyridine-3-sulfonamide;
<58> 5-chloro-N-(2,4-difluoro-3-(1-methyl-7-(methylamino)-2,4-dioxo-1,4-dihydropyrimido[4,5-d]pyrimidin-3(2H)-yl)phenyl)-2-methoxypyridine-3-sulfonamide;
<59> N-(3-(7-amino-1-cyclopropyl-4-oxo-1,4-dihydropyrimido[4,5-d]pyrimidin-3(2H)-yl)-2,4-difluorophenyl)-2,5-dichlorobenzenesulfonamide;
<60> N-(3-(7-amino-1-cyclopropyl-4-oxo-1,4-dihydropyrimido[4,5-d]pyrimidin-3(2H)-yl)-2,4-difluorophenyl)-5-chloro-2-methoxypyridine-3-sulfonamide;
<61> N-(3-(7-amino-1-cyclopropyl-4-oxo-1,4-dihydropyrimido[4,5-d]pyrimidin-3(2H)-yl)-2,4-difluorophenyl)naphthalene-1-sulfonamide;
<62> N-(3-(7-amino-1-cyclopropyl-4-oxo-1,4-dihydropyrimido[4,5-d]pyrimidin-3(2H)-yl)-2,4-difluorophenyl)-5-chloro-1,3-dimethyl-1H-pyrazole-4-sulfonamide;
<63> N-(3-(7-amino-1-cyclopropyl-2,4-dioxo-1,4-dihydropyrimido[4,5-d]pyrimidin-3(2H)-yl)-2,4-difluorophenyl)-2,5-dichlorobenzenesulfonamide;
<64> N-(3-(7-amino-1-cyclopropyl-2,4-dioxo-1,4-dihydropyrimido[4,5-d]pyrimidin-3(2H)-yl)-2,4-difluorophenyl)naphthalene-1-sulfonamide;
<65> N-(3-(7-amino-1-cyclopropyl-2,4-dioxo-1,4-dihydropyrimido[4,5-d]pyrimidin-3(2H)-yl)-2,4-difluorophenyl)-5-chloro-2-methoxypyridine-3-sulfonamide; and
<66> N-(3-(7-amino-1-cyclopropyl-2,4-dioxo-1,4-dihydropyrimido[4,5-d]pyrimidin-3(2H)-yl)-2,4-difluorophenyl)-5-chloro-1,3-dimethyl-1H-pyrazole-4-sulfonamide.

7. A method for preparing a compound represented by formula 1, comprising a step of reacting a compound represented by formula 2 with a compound represented by formula 3 to prepare a compound represented by formula 1, as shown in Reaction Formula 1 below: 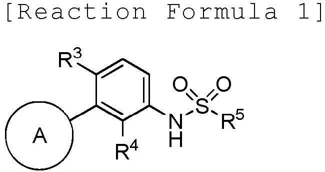
in Reaction Formula 1,
A, R¹, R², R³, R⁴, and R⁵ are as defined in Formula 1 of claim 1.

8. A pharmaceutical composition for use in preventing or treating cancer, comprising a compound represented by Formula 1 of claim 1, a stereoisomer thereof, a solvate thereof, a hydrate thereof, or a pharmaceutically acceptable salt thereof as an active ingredient.

9. A pharmaceutical composition for use in preventing or treating a disease caused by overactivation of kinases involved in ISR (integrated stress response), comprising a compound represented by Formula 1 of claim 1, a stereoisomer thereof, a solvate thereof, a hydrate thereof, or a pharmaceutically acceptable salt thereof as an active ingredient.

10. A pharmaceutical composition for use in inhibiting one or more signal transductions selected from the group consisting of HRI (heme-regulated initiation factor-2a kinase), PKR (dsRNA-activated protein kinase), PERK (PKR-like endoplasmic reticulum kinase) and GCN2 (general control nonderepressible 2), comprising a compound represented by Formula 1 of claim 1, a stereoisomer thereof, a solvate thereof, a hydrate thereof, or a pharmaceutically acceptable salt thereof as an active ingredient.

11. The pharmaceutical composition of claim 8, wherein the cancer is leukemia, lymphoma, multiple myeloma, bladder cancer, breast cancer, colon cancer, kidney cancer, liver cancer, lung cancer, small cell lung cancer, esophageal cancer, gallbladder cancer, ovarian cancer, pancreatic cancer, stomach cancer, cervical cancer, thyroid cancer, prostate cancer, or skin cancer.

12. A health functional food for use in preventing or alleviating cancer, comprising a compound represented by Formula 1 of claim 1, a stereoisomer thereof, a solvate thereof, a hydrate thereof, or a pharmaceutically acceptable salt thereof as an active ingredient.
